# FASCICULE DE BREVET EUROPEEN

(11) **EP 0 734 379 B1**
(45) Date de publication et mention de la délivrance du brevet: **07.03.2001**
(21) Numéro de dépôt: 95903385.3
(22) Date de dépôt: 09.12.1994
(51) Int. Cl.: C07D 211/14, C07D 207/06

(54) **DERIVES DE 2-ARYLALKENYL-AZACYCLOALKANES LIGANDS AUX RECEPTEURS SIGMA, LEUR PROCEDE DE PREPARATION ET LEUR APPLICATION EN THERAPEUTIQUE**
DERIVATE VON 2-ARYLALKENYL-AZACYCLO ALKAN ALS SIGMA REZEPTOR-LIGANDEN, VERFAHREN ZU IHRER HERSTELLUNG UND IHRER THERAPEUTISCHER ANWENDUNG
2-ARYLALKENYL-AZACYCLOALKANE DERIVATIVES AS SIGMA RECEPTOR LIGANDS, PREPARATION METHOD THEREFOR AND THERAPEUTICAL USE THEREOF

(30) Priorité: 09.12.1993 FR 9314814
(43) Date de publication de la demande: 02.10.1996
(60) Demande divisionnaire: 99114215.9
(73) Titulaire: PARKE-DAVIS, 92400 Courbevoie (FR)
(72) Inventeur: CALVET, Alain, Pierre, F-94240 L'Hay-les-Roses (FR); JACOBELLI, Henry, F-91550 Paray-Vieille-Poste (FR); JUNIEN, Jean-Louis, F-92310 Sèvres (FR); RIVIERE, Pierre, F-75003 Paris (FR); ROMAN, François, Joseph, F-9440 Vitry-sur-Seine (FR)
(86) Numéro de dépôt international: FR9401439
(87) Numéro de publication internationale: WO9515948

(56) Documents cités:
- EP-A- 0 362 001
- EP-A- 0 445 013
- WO-A-91/03243
- WO-A-91/06297
- WO-A-91/09594
- WO-A-92/18127
- WO-A-92/22527
- WO-A-93/09094
- WO-A-93/15052

## Description

La présente invention a pour objet des nouveaux composés dérivés de 2-arylalkényl-azacycloalkanes ligands *in vitro* aux récepteurs sigma (σ), potentiellement utiles au traitement d'affections gastro-intestinales et au traitement de dysfonctionnements neurologiques et/ou d'états psychotiques.

### Arrière-plan technologique de l'invention.

Le brevet EP 362 001 décrit des N-cycloalkylalkylamines α,α disubstituées d'affinité spécifique aux récepteurs sigma (σ), utiles au traitement des psychoses et des affections gastro-intestinales, de formule 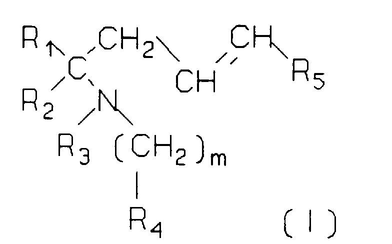 dans laquelle :
R₁ et R₅ sont phényle, R₂ est alkyle, R₃ est hydrogène ou alkyle inférieur, R₄ est cycloalkyle, m est 1 ou 2.

La demande EP 445 013 décrit des N-cycloalkylalkylamines d'affinité spécifique aux récepteurs σ, utiles au traitement des psychoses et des affections gastro-intestinales, de formule 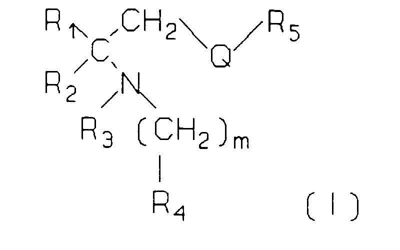 dans laquelle :
R₁ est un radical furyle ou thiényle ou encore phényle sous réserve que Q soit cyclopropane 1,2-diyle, R₂ est alkyle inférieur, R₃ est hydrogène ou alkyle inférieur, m a pour valeur 1 ou 2, R₄ est cycloalkyle -CH(CH₂)ₙ avec n de 2 à 5, R₅ est phényle ou thiényle, Q est éthylène 1,2-diyle ou cyclopropane 1,2-diyle.

Bien que présentant une affinité aux récepteurs de même type que les composés de la présente invention, ces deux documents présentent des amines différentes de par leur structure, qui est celle d'amines dans lesquelles l'atome d'azote n'est pas inclus dans une séquence cycloalkane.

A la demande WO 91/03243 il est décrit des 1-cycloalkyl-pipéridines d'activité antagoniste spécifique aux récepteurs a, utiles au traitement des psychoses et des dyskinésies, de formule 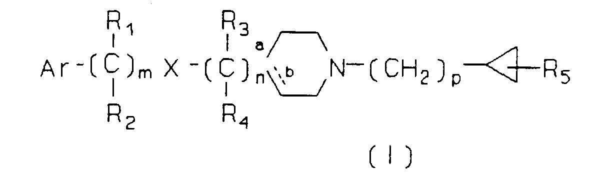 dans laquelle, de préférence :
X est C=O, CHOH ou O ; et/ou m est 0 ; et/ou n et p sont 1 ; et/ou R₃-R₅ sont H ; et/ou Ar est phényle optionnellement substitué avec des halogènes, OCH₃, NH₂, NO₂ ou un autre groupe phényle, a et b représentant par ailleurs des liaisons simples ou, soit l'un soit l'autre, une liaison double.

A la demande WO 93/09094 il est décrit des éthers dérivés d'alkyl pipéridines ou de pyrrolidines, qui sont des agents antipsychotiques de formule 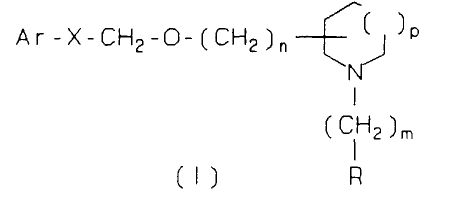 dans laquelle, pour les composés préférés :
n et p sont 1 ; et/ou m est 1-3 ; et/ou R est phényle ; et/ou X est trans -CH=CH- ; et/ou Ar est phényle, *p*-F-phényle ou *p*-CF₃-phényle ; et/ou la chaîne latérale est située en position 4 du cycle pipéridine.

Entre autres dissemblances, les composés des demandes WO 91/03243 et WO 93/09094 se différencient formellement des composés de la présente invention de par l'existence dans leur chaîne intermédiaire d'une fonction oxygénée (C=O, CHOH) ou d'un atome d'oxygène -O-. Egalement il est notable que cette chaîne est située ou annoncée préférée liée à l'atome de carbone en position 4 *(para)* du cycle pipéridine, et en aucun cas sur l'atome de carbone en position 2, adjacent à l'atome d'azote. Ces demandes ne mentionnent pas d'application des composés au traitement des affections gastro-intestinales. La demande WO 92/22527 décrit des composés antagonistes aux canaux calciques de formule 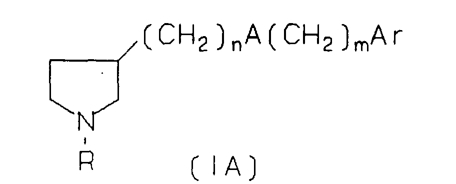 dans laquelle, entre autres :
R est C₁₋₈ alkyl C₃₋₈ cycloalkyle ; p est 0 à 2 ; n est 0 à 6 ; A est -CH=CH- ; Ar est aryle.

La demande WO 93/15052 décrit des composés antagonistes aux canaux calciques de formule 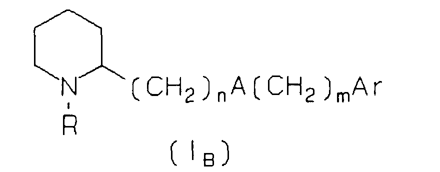 dans laquelle, Ar étant aryle ou hétéroaryle optionnellement substitué, il est défini pour les composés préférés que : m a pour valeur 0 à 3, R est C₁₋₈ alkyl(phényl)p dans lequel p est 0 ou 1, ou R est C₂₋₈ alkényl(phényl)p dans lequel p est 1, A est l'oxygène ou -CH=CH-, la longueur de la chaîne -(CH₂)ₙA-(CH₂)ₘ- étant de 2 à 6 atomes.

Ces deux dernières demandes visent des composés antagonistes aux canaux calciques, différents par cette application des composés de la présente invention. Par ailleurs, contrairement à ce que les significations annoncées pour R, A, Ar, n et m laissent supposer, aucun des composés dont il est fait mention dans ces documents ne porte atteinte à la nouveauté des 2-alkényl-azacycloalkanes (I) objets de la présente invention.

### Sommaire de l'invention.

L'invention a pour objet de nouveaux dérivés de 2-arylalkényl-azacycloalkanes de formule (I) 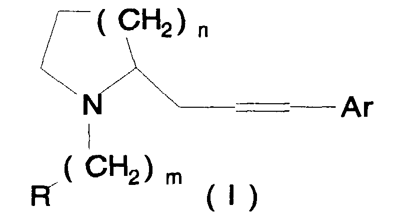 dans laquelle Ar est aryle ou hétéroaryle optionnellement mono à trisubstitué,
m a pour valeur 1 ou 2,
R est phényle optionnellement substitué, ou cycloalkyle comprenant de 3 à 7 atomes de carbone,
n a pour valeur 1 à 3,
leurs isomères, leurs dérivés dans lesquels un atome est remplacé par un de ses isotopes radioactifs, et leurs sels d'addition avec les acides pharmaceutiquement acceptables. Les composés de l'invention montrent une affinité particulièrement intéressante *in vitro* pour les récepteurs σ, qui est significative de leur utilité à la prévention ou au traitement de dysfonctionnements neurologiques et/ou d'états psychotiques ; et, *in vivo,* ils montrent une activité pharmacologique particulièrement significative de leur utilité au traitement d'affections gastro-intestinales.

Les composés préférés de l'invention sont ceux dans lesquels :
Ar est un radical phényle optionnellement substitué,
n a pour valeurs 1 ou 2,
R est un radical cyclopropyle, cyclobutyle, ou phényle.

La présente invention rapporte également le procédé de préparation des azacycloalkanes (I) ainsi que des compositions pharmaceutiques comprenant un composé (I), associé à des excipients, diluants ou solvants pharmaceutiquement acceptables.

### Description détaillée de l'invention

En ce qui concerne les composés (I) de la présente invention :
- par aryle, il est entendu des radicaux carbonés insaturés mono ou bicycliques comme phényle, indényle, naphtyle, pouvant être partiellement saturés comme indanyle ou tétrahydronaphtyle. Le radical phényle, optionnellement mono à trisubstitué, est préféré. Dans ce cas, les substituants, identiques ou différents en cas de plurisubstitution, sont choisis dans l'ensemble comprenant des halogènes, le groupe nitro, des radicaux alkyle inférieur, haloalkyle inférieur, alkoxy inférieur, haloalkoxy inférieur, dans lesquels par inférieur on entend des chaînes carbonées comprenant de 1 à 4 atomes de carbone. On préfère particulièrement le radical phényle non substitué ou mono ou disubstitué par des atomes d'halogène et/ou des radicaux alkoxy inférieur, comme de préférence, méthoxy.
- par hétéroaryle, on entend des radicaux mono ou bicycliques insaturés comprenant un ou deux hétéroatomes choisis parmi l'oxygène, l'azote, le soufre. Les hétérocycles azotés et/ou soufrés sont préférés, notamment les radicaux pyridinyle ou thiényle.

En ce qui concerne les isomères, ceux-ci comprennent à la fois les isomères géométriques consécutifs à la liaison π de la séquence arylalkényle, et les isomères optiques consécutifs à l'asymétrie du carbone en position 2 du cycle azacycloalkane ainsi que leurs mélanges, et en particulier le mélange racémique.

En ce qui concerne les sels d'addition des composés (I), par acides pharmaceutiquement acceptables on entend des acides minéraux ou organiques démontrés atoxiques aux doses thérapeutiques courantes. Ce sont, pour exemples non limitatifs, les acides acétique, benzènesulfonique, camphosulfonique, citrique, éthanesulfonique, bromhydrique, lactique, maléique, malique, méthanesulfonique, mucique, nitrique, pamoïque, phosphorique, salicylique, stéarique, succinique, sulfurique, tartrique, et l'acide chlorhydrique qui est préféré. On trouvera une revue des sels acceptables en pharmacie dans *J*. *Pharm. Sci.,* 1977, vol. 66, p. 1- 19.

Par un autre aspect, l'invention concerne un procédé de préparation des composés (I), montré au schéma 1, à partir d'un azacycloalkane (III) 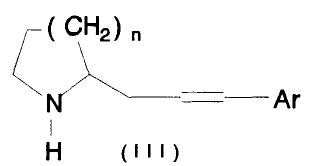 dans lequel n et Ar sont tels que définis pour (I), qui consiste :
- soit à alkyler un intermédiaire (III) par un halogénure d'alkyle (IV') 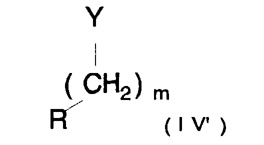 dans lequel m et R sont tels que définis pour (I), et Y est un halogène, de préférence le chlore ou le brome,
- soit, de la façon préférée, à acyler l'intermédiaire (III) par un réactif (IV) 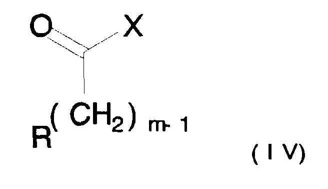 dans lequel m et R sont tels que défini pour (I), et X est -OH ou un halogène comme le chlore ou le brome, pour obtenir un dérivé carboxamide intermédiaire (II) 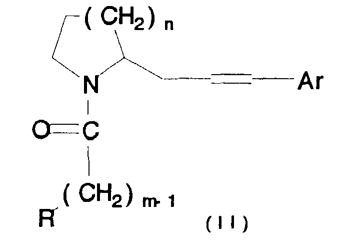
que l'on réduit par un hydrure métallique ou organométallique dérivé du bore ou de préférence de l'aluminium.

De façon plus précise, la mise en oeuvre du procédé d'alkylation de l'intermédiaire (III) par l'halogénure (IV') qui, de préférence, est un chlorure ou un bromure d'alkyle, est réalisée dans un solvant inerte tel que le toluène ou l'acétonitrile. Eventuellement, il est ajouté au milieu réactionnel un agent basique, minéral comme le carbonate de sodium, ou organique comme la triéthylamine, afin de favoriser la réaction. Pour 1 mol d'intermédiaire (III) engagé on peut utiliser de 0,5 à 1,5 mol d'halogénure d'alkyle, la réaction étant réalisée dans 2 à 3 1 du solvant choisi. Selon les réactifs on obtient un résultat satisfaisant après une durée de réaction comprise entre 1 à 24 h pour des conditions de température comprises entre 20 et 110 °C.

Le procédé de préparation préféré consiste dans une première étape à obtenir à partir de l'intermédiaire (III) un carboxamide (II) puis à le réduire par un hydrure métallique ou organométallique ; lorsque, dans le réactif (IV), X est un halogène comme le chlore ou le brome, la réaction, effectuée dans le toluène ou de préférence le dichlorométhane, consiste à ajouter dans une solution contenant 1 mol de (III) entre 1,0 et 1,5 mol d'une amine organique comme la triéthylamine, puis le réactif (IV) en quantité équimoléculaire à l'amine. La solution est ensuite maintenue de 3 à 48 h à une température comprise entre 0 et 30 °C selon la nature des réactifs.

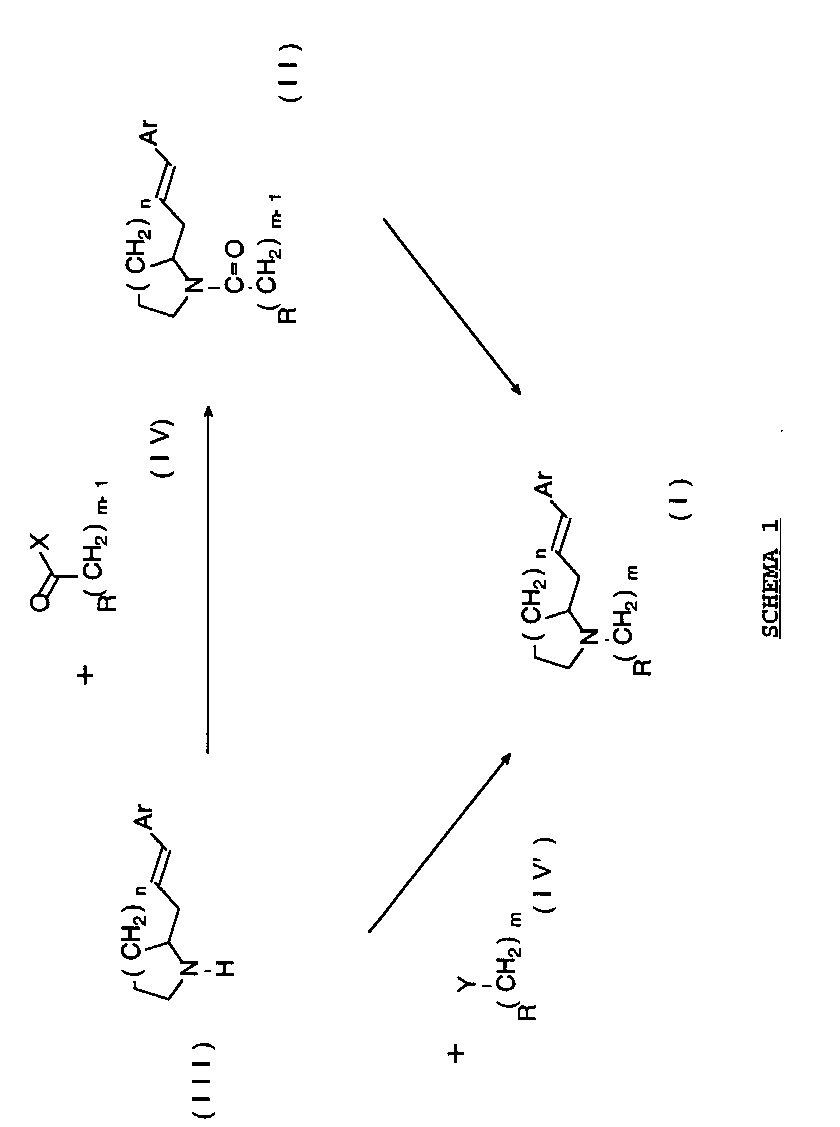

Lorsque dans le réactif (IV) X est -OH, une méthode appropriée consiste à préparer *in situ* un anhydride de l'acide, éventuellement mixte, puis à pratiquer l'acylation de (III) par cet anhydride. Favorablement on opère dans des solvants apolaires anhydres de la classe des éthers-oxydes comme le tétrahydrofurane (THF) qui est préféré. Dans un premier temps l'anhydride mixte est préparé à une température comprise entre - 40 et 0 °C en ajoutant pour 1 mol d'acide (IV), de 1,0 à 1,5 mol d'une amine tertiaire comme la N-méthylmorpholine, puis de 0,9 à 1,2 mol de chloroformiate d'isobutyle. On ajoute ensuite 1 mol de l'intermédiaire (III) et laisse la réaction se développer de 1 à 48 h à une température comprise entre 0 et 60 °C. Un résultat satisfaisant est généralement obtenu après une durée de 10 à 20 h pour une température comprise entre 10 et 25 °C.

Des méthodes alternatives utilisent des agents de déshydratation. Elles sont entre autres énumérées dans March, J. *Advanced Organic Chemistry,* 3rd ed., New-York : Wiley-Interscience, 1985, p. 372 ; celles qui consistent à utiliser le dicyclohexyl-carbodiimide ou le N-N'-carbonyl-diimidazole sont particulièrement adaptées.

La seconde étape, qui consiste à réduire le carboxamide (II), fait intervenir des hydrures métalliques ou organométalliques dérivés du bore ou de l'aluminium. Parmi ceux-ci, on peut utiliser le borane (BH₃) sous forme de complexes et, de préférence, les hydrures dérivés de l'aluminium parmi lesquels on cite à titre d'exemples des hydrures simples comme AlH₃ ou le Dibal [(CH₃)₂-CH-CH₂]₂AlH, des hydrures mixtes d'aluminium et de métaux alcalins comme le sodium ou le lithium, l'hydrure de lithium aluminium (LiAlH₄ ou LAH) et l'hydrure d'aluminium AlH₃ étant les agents réducteurs préférés.

Les réactions sont effectuées dans des solvants de la classe des éthers-oxydes comme l'éther diéthylique, le 1,2-diméthoxyéthane, et plus particulièrement le tétrahydrofurane (THF) qui est préféré notamment pour les réductions par l'hydrure d'aluminium, qui est la méthode favorablement pratiquée pour réduire les carboxamides (II). A cet effet l'hydrure est préparé in *situ* à partir d'halogénures d'aluminium et d'hydrures métalliques comme décrit par exemple dans Gaylord, N.J. *Réduction with complex metal hydrides,* ed: Interscience, 1956, p. 6-8, et p. 51- 53.

Dans les conditions préférées, la réduction dans le THF d'une mole de carboxamide (II) consiste d'abord à générer *in situ* l'hydrure d'aluminium par réaction de 0,75 à 2 mol de AlCl₃ avec 2,2 à 6 mol de LAH, ces deux réactifs étant dans un rapport stoechiométrique de 1 pour 3, puis à procéder à la réduction de (II), que l'on ajoute à une température comprise entre - 10 et + 30 °C. La réaction est maintenue de 1 à 24 h à la même température, puis on décompose les complexes obtenus et on isole les composés de l'invention (I) par les méthodes appropriées habituelles à l'homme de l'art. Des résultats satisfaisants sont habituellement obtenus pour ces réductions après 2 à 6 h de réaction à une température entre 10 et 20 °C. Telle que décrite, la préparation des composés (I) fait appel aux azacycloalkanes intermédiaires essentiels (III) qui sont préparés par des méthodes décrites ou adaptées de la littérature, à partir soit de 2-(2-hydroxyéthyl)-azacycloalkanes (VIII), soit de N-nitroso-azacycloalkanes (X), tel qu'il est montré au schéma 2.

Le procédé de préparation des azacycloalkanes (III) à partir de 2-(2-hydroxyéthyl)-azacycloalkanes (VIII), lesquels sont accessibles dans le commerce ou préparés selon des procédés de l'état de la technique, consiste à préparer dans un premier temps un carbamate intermédiaire (VII), dans lequel Z est alkyle, aryle, ou encore polyalkylaryle, le radical *t*-butyle étant toutefois préféré dans un groupement protecteur N-*t*-butyloxycarbonyle (N-Boc). La mise en oeuvre de ces préparations fait appel à des conditions largement décrites comme par exemple par Geiger, R. et Koenig, W. In Gron, E. et Meienhofer, J. The *peptides.* New-York : Academic Press, 1980, vol. 3, p. 3-136. L'agent préféré est le di-*tert*-butyl dicarbonate, que l'on fait réagir en léger excès sur le composé (VIII) en solution dans le dichlorométhane, à une température comprise entre 0 et 30 °C.

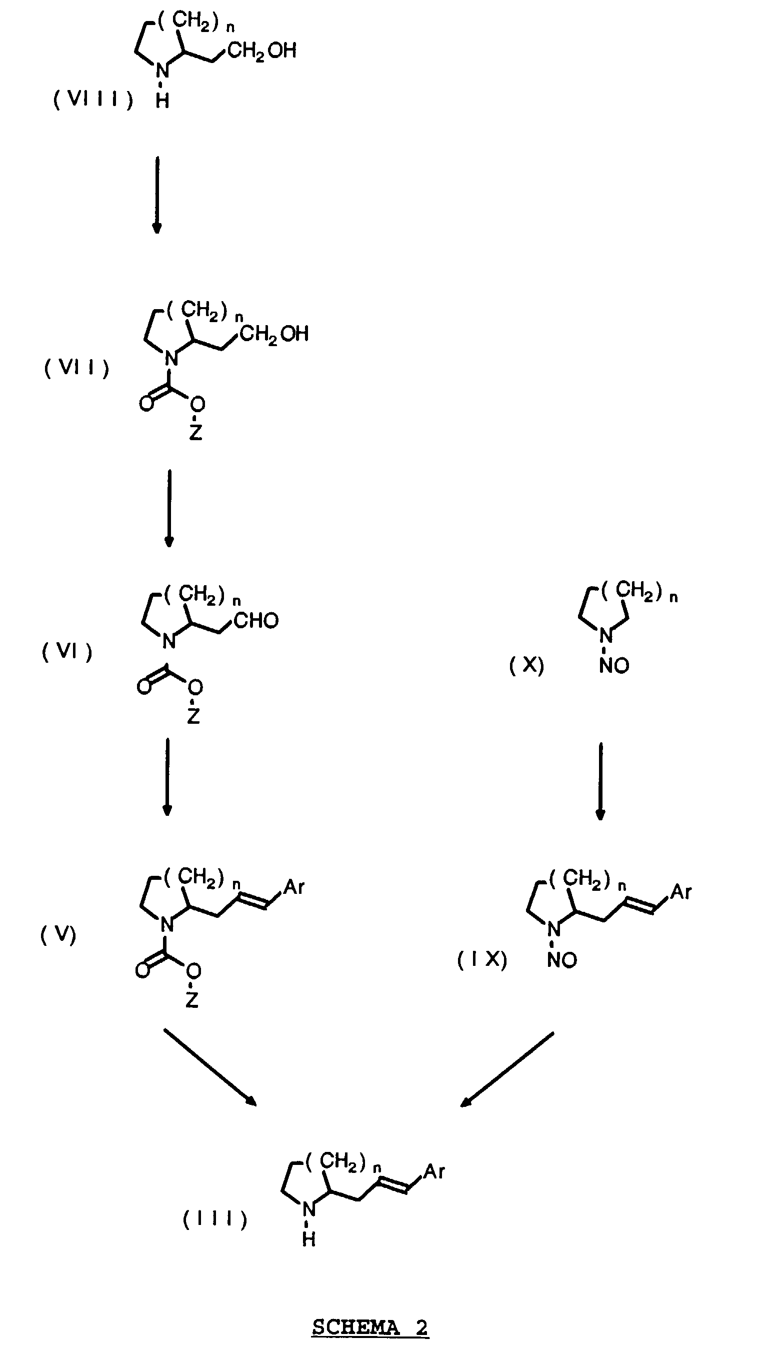

On procède ensuite à l'oxydation ménagée du carbamate (VII). Le réactif utilisé à cet effet peut être choisi parmi ceux décrits par exemple dans March, J. *Advanced Organic Chemistry.* 3rd ed. p. 1057- 1060. Le réactif préféré est le chlorochromate de pyridinium que l'on utilise en milieu éthéré, ou dans le nitrobenzène, la pyridine, ou encore dans un hydrocarbure halogéné comme le dichlorométhane, qui est préféré. Couramment pour 1 mol de composé (VII) à oxyder, on utilise de 1,5 à 4 mol de chlorochromate de pyridinium à une température comprise entre 0 et 40 °C durant 8 à 30 h, pour obtenir l'aldéhyde intermédiaire (VI).

Ce composé est alors engagé dans une réaction de Wittig avec un halogénure de triphénylphosphonium de formule Ar-CH₂-P≡(C₆H₅)₃⁺ Hal⁻, dans lequel Ar est tel que défini pour (I) et Hal représente un halogène comme le chlore, le brome ou l'iode. Différentes mises en oeuvre des réactifs sont décrites comme par exemple dans *Organic Reactions,* vol. 14, p. 270. Elles font appel à des réactifs basiques, et peuvent être réalisées en milieux hétérogènes biphasiques. Le procédé utilisé consiste toutefois à réaliser la réaction dans un alcool comprenant jusqu'à 3 atomes de carbone et en présence d'alkoxyde de sodium qui est formé *in situ.* L'éthanol est préféré, et on utilise des quantités voisines de la stoechiométrie pour le dérivé de triphénylphosphonium et pour l'agent alcalin, la réaction étant réalisée à une température comprise entre 10 et 50 °C, pour obtenir l'intermédiaire N-carbamyl-2-arylalkényl-azacycloalkane (V), qui est soumis à une réaction de N-déprotection par l'acide trifluoroacétique par exemple, pour obtenir l'intermédiaire azacycloalkane essentiel (III) sous forme d'un mélange des isomères Z et E. Ceux-ci sont séparés par des techniques classiques, notamment par chromatographie sur colonne de silice, ainsi que par cristallisation sélective du chlorhydrate. L'identification des isomères Z ou E est effectuée par RMN du proton ; en effet, dans -CHₐ=CH_{b}-, la constante de couplage entre Hₐ et H_{b} vaut typiquement 10 Hz si la molécule est Z, et 17 Hz environ si elle est E (Silverstein R.M. et coll. *Spectrometric identification of organic compounds,* 4th ed., New York : Wiley, 1981, p. 235).

Le procédé de préparation alternatif des composés (III) est à conduire avec prudence, notamment pour des opérations de volume important, car il fait appel à des réactifs et intermédiaires dangereux ou annoncés potentiellement carcinogènes. Il consiste à pratiquer l'alkylation de N-nitroso-azacycloalkanes (X), accessibles dans le commerce ou préparés par réaction de nitrosation d'azacycloalkane, avec un agent d'alkylation Ar-CH=CH-CH₂-Hal, dans lequel l'halogène est le chlore ou le brome, pour obtenir un intermédiaire N-nitroso-2-arylalkénylazacycloalkane (IX), que l'on soumet à une réaction de N-dénitrosation pour obtenir l'intermédiaire essentiel (III). Dans le cadre de la description expérimentale de l'invention, ce procédé est pratiqué sur de faibles quantités, selon le mode opératoire décrit par Seebach, D. et Enders, D. *Chem. Ber.,* 1975, vol. 108, p. 1293- 1320.

Pour préparer un composé (III) optiquement actif, on peut :
- condenser un composé (III) racémique avec un dérivé d'acide α-aminé appartenant à la série D ou à la série L et dans lequel la fonction amine est protégée. Après déprotection, le produit obtenu est séparé en ses diastéréoisomères par chromatographie ; par dégradation d'Edman on revient ensuite avec deux énantiomères de l'amine (III) ; ou encore,
- dissoudre un composé (III) racémique dans une solution d'acide optiquement actif, par exemple un énantiomère de la N-acétyl-phénylalanine, pour former deux sels diastéréoisomères, puis par différence de solubilité, en cristalliser sélectivement un dans un solvant approprié.

La présente invention est illustrée de façon non limitative par les exemples qui suivent. L'état de pureté, les caractéristiques physico-chimiques et l'identité structurelle des produits sont déterminés et rapportés comme suit :
- les produits sont purifiés par des techniques appropriées, notamment par la chromatographie sur colonne pour laquelle on utilise favorablement la technique dite de chromato-flash sur colonne de silice (fournisseur Merck, produit Kieselgel H 60, granulométrie 230 à 400 mesh). L'état de pureté des produits obtenus est déterminé par la méthode de chromatographie sur couche mince (CCM) de silice (plaques prêtes à l'emploi Merck) ; les Rf observés ainsi que les solvants d'élution utilisés sont indiqués dans les exemples.
- les caractéristiques physico-chimiques des produits, sont représentées :
   a) par le point de fusion, déterminé par la méthode du tube capillaire et dont la valeur indiquée n'est pas corrigée ;
   b) par la spectrographie infrarouge (IR) des composés en pastilles de KBr ; les absorptions les plus intenses sont rapportées par la valeur de leur longueur d'onde en cm⁻¹ ;
   c) par le pouvoir rotatoire, déterminé à une température voisine de 20 °C sur un appareil Polartronic en cuve de 10 cm de long ;
- l'identité structurelle des produits est déterminée en accord avec :
   a) la résonance magnétique nucléaire du proton (RMN) étudiée à 90 ou à 400 MHz, les produits étant solubilisés dans le deutérochloroforme. L'aspect des signaux et leur déplacement chimique exprimé en p.p.m. par rapport au tétraméthylsilane utilisé comme référence interne sont indiqués. Les protons dits échangeables après addition d'oxyde de deutérium sont également signalés ;
   b) l'analyse centésimale élémentaire, dont les résultats conformes aux normes admises ne sont pas reportés ; cependant ces analyses sont signalées être effectuées par la représentation de l'élément dosé ;
   c) la chromatographie liquide à haute pression sur colonne chirale Alpha AGP (α-glyco-protéine), avec détection UV à 220 nm, qui permet d'apprécier la pureté optique.

### Partie expérimentale chimique

### Préparation 1 : E-2-cinnamyl-pyrrolidine (f. III ; Ar = C₆H₅, n = 1)

- Stade a) Dans un réacteur d'un litre, protégé de l'humidité et sous atmosphère d'azote on introduit 600 ml de THF déshydraté sur tamis moléculaire et 25,3 g (35,0 ml - 0,25 mol) de diisopropylamine. La solution est refroidie à - 70 °C par un mélange de carboglace-acétone et on ajoute en maintenant à - 60 / - 50 °C 100 ml d'une solution 2,5 M (0,25 mol) de n-butyl lithium dans l'hexane.
   Le mélange est maintenu 15 min sous agitation à - 70 °C puis on ajoute en 5 min à cette même température une solution de 25,0 g (0,25 mol) de N-nitrosopyrrolidine (f. X ; n = 1) dans 25ml de THF anhydre. La solution orangée est agitée 10 min puis on ajoute en 15 min à - 70 °C 49,3 g (0,25 mol) de bromure de cinnamyle en solution dans 50 ml de THF anhydre.
   Le milieu réactionnel est maintenu 2 h à - 70 °C, puis sous agitation 16 h à - 20 / - 25 °C, après quoi il est introduit 15,0 ml d'acide acétique pur, ce qui provoque la formation d'un insoluble jaunâtre. La suspension est précipitée sous agitation dans 600 ml de solution saturée en NaCl et 600 ml de dichlorométhane. La phase aqueuse est séparée, extraite par 200 ml de dichlorométhane. Les phases organiques réunies sont extraites par 200 ml de solution saturée en NaCl puis déshydratées sur Na₂SO₄.
   Après évaporation sous vide et sur bain-marie du solvant, l'huile marron résiduelle (60,0 g) est purifiée par chromatographie sur colonne de silice. L'élution par le dichlorométhane permet d'obtenir la E-2-cinnamyl-N-nitrosopyrrolidine (f. IX ; Ar = C₆H₅, n = 1), purifiée sous forme d'une huile visqueuse jaunâtre.
   - Poids :: 24,9 g Rdt : 46 %
   - - CCM :: Rf = 0,75- 0,85 (hexane/acétate d'éthyle 50/50 v/v)
   - - RMN :: 1,80 - 2,40 (m, 4H) ; 2,40 - 3,20 (m, 2H) ; 3,30 - 3,90 (m, 2H) ; 4,25 - 4,70 (m, 1H) ; 6,00 - 6,60 (m, 2H) ; 7,10- 7,50 (m, 5H).
- Stade b) Dans un réacteur protégé de l'humidité, on introduit en solution dans 1200 ml d'éther diéthylique anhydre 24,0 g (0,111 mol) de dérivé N-nitroso obtenu au stade a) précédent. Sous agitation et en maintenant une température de 25 ± 5 °C la solution est saturée en 1 h 30 min environ par barbotage d'acide chlorhydrique gazeux. La solution est abandonnée 16 h sous agitation à 15- 20 °C, puis l'excès d'acide est éliminé par barbotage d'azote.
   La phase éthérée est extraite par 3 x 800 ml d'eau. Les phases aqueuses acides réunies sont alcalinisées à une température inférieure à 10 °C par addition de solution d'hydroxyde de sodium jusqu'à pH 12. Le mélange est extrait par 3 x 750 ml d'éther ; les phases éthérées réunies sont lavées par extractions avec 3 x 400 ml de solution aqueuse saturée en NaCl, puis séchées sur Na₂SO₄. Après évaporation de l'éther, le produit brut (13,6 g) est purifié par chromatographie sur colonne de silice.
   L'élution par le dichlorométhane puis le mélange dichlorométhane/méthanol ammoniacal à 10 % 92/8 v/v permet d'obtenir le produit purifié sous forme d'huile visqueuse jaune pâle.
   - Poids :: 9,0 g Rdt : 43 %
   - - CCM :: 0,35- 0,45 (dichlorométhane/méthanol ammoniacal à 10 % 92/8 v/v)
   - - RMN :: 1,20- 2,10 (m, 4H) ; 2,35 (t, 2H) ; 2,70- 3,20 (m, 3H) ; 3,3 (s, 1H éch. D₂O) ; 6,00- 6,60 (m, 2H) ; 7,00- 7,50 (m, 5H).
- Stade c) (-)E-2-cinnamyl-pyrrolidine
   On dissout 13,5 g (0,072 mmol) de (+/-)-E-2-cinnamyl-pyrrolidine obtenue au stade a) précédent et 7,46 g (0,036 mmol) de N-acétyl-phénylalanine (L) dans 250 ml d'acétone à ébullition. Après filtration sur terre d'infusoires, l'insoluble qui précipite par refroidissement 16 h à 20 ± 3 °C est filtré, puis recristallisé de la même manière à deux reprises. Par traitement en milieu alcalin, on obtient, après extraction à l'éther puis évaporation, la (-)-E-2-cinnamyl-pyrrolidine, sous forme d'une huile jaune pâle.
   Poids 2,5 g [α]_{D} = - 12,8 ° (c = 1, dichlorométhane)
- Stade d) (+)-E-2-cinnamyl-pyrrolidine
   Le filtrat obtenu lors de la première filtration du stade c) précédent est traité en milieu alcalin ; on obtient 9,5 g d'une base enrichie en énantiomère (+). Comme au stade précédent, on prépare dans l'acétone un sel diastéréoisomère, cette fois avec la N-acétyl-phénylalanine (D). On obtient 2,6 g de (+)-E-2-cinnamyl-pyrrolidine. [α]_{D} = + 13,6 ° (c = 1, dichlorométhane)

### Préparations 2

### Intermédiaires (VI)

### 1°) N-t-butyloxycarbonyl-2-(2-acétaldéhyde)-pipéridine (f. VI ; n = 2)

- Stade 1 : Dans un réacteur de 2 1 protégé de l'humidité, on introduit 40,0 g (0,309 mol) de 2-(2-hydroxyéthyl)-pipéridine (f. VIII ; n = 2) dans 600 ml de dichlorométhane déshydraté sur tamis moléculaire. A la solution jaune pâle obtenue on ajoute rapidement 80,0 g (0,370 mol) de di-*tert*-butyl dicarbonate. On laisse agiter une heure à 20- 25 °C puis abandonne 16 h à 20 °C.
   Le solvant est éliminé par distillation sous vide et sur bain-marie. L'huile jaune résiduelle est purifiée par chromatographie sur colonne de silice. L'élution par le mélange dichlorométhane/méthanol 95/5 v/v permet d'obtenir le N-*t*-butyloxy-carbonyl-2-(2-hydroxyéthyl)-pipéridine (f. VII ; Z = *t*-butyle, n = 2).
   - Poids :: 66,7 g Rdt : 94 %
   - - CCM :: Rf = 0,70- 0,80 (acétate d'éthyle/hexane 90/10 v/v)
   - - RMN :: 1,20- 2,20 (m, 18H) ; 2,50- 3,00 (m, 1H) ; 3,20- 3,80 (m, 2H) ; 3,80- 4,50 (m, 2H).
   - Stade 2 : Dans un réacteur de 3 1 protégé de l'humidité, on dissout dans 2,4 1 de dichlorométhane anhydre 66,0 g (0,287 mol) de l'alcool N-protégé obtenu au stade a) précédent. On ajoute à la solution 125,0 g (0,58 mol) de chlorochromate de pyridinium. La suspension orangée qui devient rapidement noirâtre est maintenue 16 h à 20 ± 3 °C sous agitation.
   Après quoi, la phase organique est décantée, extraite par 1 l de solution NaOH 1 N. Le mélange émulsionné est filtré sur un Buchner garni de terre d'infusoires. Le filtrat est décanté, la phase aqueuse est écartée. La phase organique est déshydratée sur Na₂SO₄, puis le solvant éliminé par distillation. Le résidu noirâtre (35,0 g) est purifié par chromatographie sur colonne de silice. L'élution par l'acétate d'éthyle permet d'obtenir la N-*t*-butyloxycarbonyl-2-(2-acétaldéhyde)-pipéridine (f. VI ; Z = *t*-butyle, n = 2).
   - Poids :: 16,2 g Rdt : 25 %
   - - CCM :: Rf = 0,50- 0,60 (dichlorométhane/éther diéthylique 50/50 v/v)
   - - RMN :: 1,20- 2,00 (m, 15H) ; 2,35- 3,00 (m, 3H) ; 3,80- 4,20 (m, 1H) ; 4,70- 5,00 (m, 1H) ; 9,70- 9,80 (m,1H).

### 2°) N-t-butyloxycarbonyl-2-(2-acétaldéhyde)-pyrrolidine (f. VI; n = 1)

- stade 1 : N-Boc-2-pyrrolidine méthanol
   Le composé est préparé tel que décrit au § 1°), stade 1 précédent, à partir de 2-pyrrolidine éthanol, avec un rendement de 100 %.
   - - CCM :: Rf = 0,70- 0,85 (acétate d'éthyle/hexane 95/15 v/v)
- Stade 2 : N-Boc-2-pyrrolidine méthanal
   Le composé est préparé à partir de l'alcool N-protégé obtenu au stade 1 précédent selon le mode opératoire décrit au § 1°), stade 2, avec un rendement de 68 %.
   - - CCM :: Rf = 0,85- 0,95 (acétate d'éthyle/hexane 80/20 v/v)
- Stade 3 : N-Boc-2-(2-méthoxy-éthényl)-pyrrolidine
   Dans un réacteur protégé de l'humidité, on introduit 895 ml d'éthanol absolu puis, sous agitation, 11,8 g (0,510 mol) de sodium décapé. Après dissolution, à 20- 25 °C, on ajoute 174,7 g de chlorure de (méthoxy-méthyl)-triphényl-phosphonium. La suspension blanche est agitée 30 min à 20- 25 °C puis on introduit une solution de 71 g (0,356 mol) de l'aldéhyde obtenu au stade 2 précédent, dans 199 ml d'éthanol absolu. Le milieu réactionnel est maintenu 2 h 30 min à ébullition, puis refroidi et évaporé sous vide et sur bain-marie. Le résidu orange est repris dans le pentane puis filtré. Le filtrat est concentré et chromatographié sur silice en éluant par le mélange hexane/acétate d'éthyle 85/15 v/v. Poids = 60 g Rdt : 74 %.
   - - CCM :: Rf = 0,75- 0,90 (hexane/acétate d'éthyle 70/30 v/v)
- Stade 4 : Dans un réacteur, on introduit 1 1 de tétrahydrofurane et 102 g de l'éther vinylique obtenu au stade précédent, puis 150 ml d'acide chlorhydrique à 10 % (p/v). La solution marron est maintenue sous agitation à 40 °C pendant 30 min, puis refroidie. On ajoute 1 l d'éther et on décante la phase organique qui est lavée par une solution saturée de NaCl, puis évaporée. Le résidu marron est purifié par chromato-flash sur silice en éluant par le mélange hexane/acétate d'éthyle 75/25 v/v.
   - Poids :: 71,9 g Rdt : 75 %.

### Préparation 2A : Z et E 2-cinnamyl-pipéridine (f. III ; Ar = C₆H₅, n = 2)

- Stade a) Dans un réacteur protégé de l'humidité, on introduit 180 ml d'éthanol absolu puis, sous agitation, 1,63 g (0,071 mol) de sodium décapé. Après dissolution, à 20- 25 °C, on ajoute 27,3 g (0,071 mol) de chlorure de benzyl-triphénylphosphonium. La suspension jaunâtre est maintenue sous agitation 30 min à 20- 25 °C puis on introduit en 2 min environ une solution de 16,0 g (0,070 mol) de l'acétaldéhyde (f. VI ; n = 2) obtenu précédemment, dans 35 ml d'éthanol absolu. La solution blanche obtenue est maintenue 30 min à 20- 25 °C, l'insoluble est filtré sur buchner et écarté. Le filtrat est évaporé sous vide et sur bain-marie. Le résidu huileux est concrétisé dans 500 ml de *n*-pentane, ce nouvel insoluble est filtré et éliminé. Le filtrat est concentré. On obtient 19,0 g de N-*t*-butyloxycarbonyl-2-cinnamyl-pipéridine brute (f. V ; Z = *t*-butyle, Ar = C₆H₅, n = 2) (Rdt = 90 %) qui est engagée telle quelle au stade suivant.
- Stade b) Dans un réacteur protégé de l'humidité on dissout dans 400 ml de dichlorométhane anhydre 19,0 g (0,063 mol) du composé (V) obtenu au stade précédent. La solution est refroidie par un bain de glace et on ajoute sous agitation en 10 min, à une température inférieure à 5 °C, 190 ml d'acide trifluoro-acétique pur. La solution est maintenue 30 min à cette température puis concentrée sous vide et sur bain-marie. Le résidu est dissous dans 600 ml d'éther, extrait par 200 ml de solution NaOH 1 N. La phase éthérée est lavée à l'eau puis séchée sur Na₂SO₄.
   L'éther est évaporé, on obtient le produit brut (12,0 g) sous forme d'une huile marron clair que l'on dissout dans 120 ml de dichlorométhane anhydre. A cette solution, on ajoute 25 ml d'éther chlorhydrique 5 N puis évapore les solvants par distillation. Le résidu est dissous dans 150 ml d'isopropanol à ébullition. L'insoluble qui précipite par refroidissement sous agitation est filtré sur buchner puis recristallisé dans l'éthanol absolu. On obtient après élimination sous vide des solvants résiduels le chlorhydrate de E-2-cinnamyl-pipéridine (f. III- E ; Ar = C₆H₅, n = 2)
   - Poids :: 4,6 g Rdt : 30,7 % F : 225 °C
   - - CCM :: Rf = 0,30- 0,40 (dichlorométhane/méthanol ammoniacal à 10 % 90/10 v/v)
   - - Analyse (C₁₄ H₂₀ Cl N) :: % C, H, Cl, N conformes.

   Par traitement en milieu alcalin de 4,0 g (16,8 mmol) du chlorhydrate, on obtient après extraction à l'éther puis évaporation 3,3 g de E-2-cinnamyl-pipéridine sous forme d'une huile visqueuse jaune pâle.
   - - RMN :: 1,00- 1,90 (m, 6H) ; 1,95 (s, 1H éch. D₂O) ; 2,10-2,40 (m, 2H) ; 2,40- 2,80 (m, 2H) ; 2,90- 3,20 (m, 1H) ; 6,00-6,60 (m, 2H) ; 7,10- 7,45 (m, 5H).

   Le filtrat isopropanolique obtenu précédemment est concentré par distillation. Le résidu (9,0 g) est traité et extrait en milieu alcalin par l'éther. L'éther est éliminé et le résidu huileux (6,4 g) purifié par chromatographie sur colonne de silice. L'élution par le mélange dichlorométhane/méthanol ammoniacal à 10 % 95/5 v/v permet d'obtenir la Z-2-cinnamyl-pipéridine (f. III- Z ; Ar = C₆H₅, n = 2) pure, sous forme d'une huile très visqueuse qui se solidifie lentement à froid.
   - Poids :: 3,0 g Rdt : 23,7 %
   - - CCM :: Rf = 0,40- 0,50 (dichlorométhane/méthanol ammoniacal à 10 % 90/10 v/v)
   - - RMN :: 1,00- 1,90 (m, 6H) ; 2,20 (s, 1H éch. D₂O) ; 2,30-2,80 (m, 4H) ; 2,90- 3,20 (m, 1H) ; 5,50- 5,85 (m, 1H) ; 6,40- 6,60 (m, 1H) ; 7,10- 7,40 (m,5H).
- Stade c (-)-E-2-cinnamyl-pipéridine.
   A partir de (+/-)-E-2-cinnamyl-pipéridine obtenue au stade précédent, selon un mode opératoire similaire à la préparation 1 stade c) dans lequel la dernière recristallisation est conduite en milieu aqueux, on obtient avec un rendement de 33 % la (-)-E-2-cinnamyl-pipéridine, sous forme d'une huile. [α]_{D} = -8,1 ° (c = 2, dichlorométhane)
- Stade d) (+)-E-2-cinnamyl-pipéridine.
   Selon le procédé décrit à la préparation 1 stade d) en effectuant la dernière recristallisation en milieu aqueux, on obtient après retour à la base la (+)-E-2-cinnamyl-pipéridine, avec un rendement de 30 %.
   [α]_{D} = + 8,1 ° (c = 2, dichlorométhane).

### Préparation 2B : Z et E 2-(p-fluoro-cinnamyl)-pipéridine (f. III ; Ar = p-fluoro-phényle, n = 2)

- Stade a) Selon le mode opératoire de la préparation 2A, stade a), à partir du chlorure de p-fluoro-benzyl-triphényl-phosphonium, on obtient avec un rendement de 96 % la N-t-butyloxycarbonyl-2-(p-fluoro-cinnamyl)-pipéridine, sous forme d'une huile jaune.
- Stade b) La déprotection par l'acide trifluoroacétique est pratiquée selon le mode opératoire de la préparation 2A, stade b). Le produit brut obtenu sous forme d'une huile jaune orangée est un mélange des isomères Z et E que l'on sépare par chromato-flash sur colonne de silice. L'élution par le mélange dichlorométhane/méthanol ammoniacal à 10 % 95/5 v/v puis 90/10 v/v permet d'obtenir successivement différentes fractions. Aux fractions les moins polaires, reprises dans le dichlorométhane, on ajoute de l'éther chlorhydrique 5N, puis on évapore les solvants, et le résidu est dissous dans l'isopropanol, et concrétisé sous agitation par addition d'éther. On obtient un précipité blanc de chlorhydrate de Z-2-(p-fluoro-cinnamyl)-pipéridine. F = 135 °C
   Par traitement en milieu alcalin, extraction à l'éther et évaporation, on obtient la Z-2-(*p*-fluoro-cinnamyl)-pipéridine sous forme d'une huile jaune. Rdt : 13 %
   - - CCM :: Rf = 0,70- 0,85 (dichlorométhane/méthanol ammoniacal à 10 % 80/20 v/v)

   Par un traitement comparable des fractions les plus polaires, on obtient le chlorhydrate de E-2-(*p*-fluoro-cinnamyl)-pipéridine, sous forme d'un précipité blanc.
   F = 193 °C (isopropanol).
   Le retour à la base fournit la E-2-(*p*-fluoro-cinnamyl)-pipéridine avec un rendement de 25 %.
   - - CCM :: Rf = 0,60- 0,75 (dichlorométhane/méthanol ammoniacal à 10 % 80/20 v/v)

### Préparation 2C : E-2-(p-chloro-cinnamyl)-pipéridine (f. III ; Ar = p-chloro-phényle, n = 2)

- Stade a) Selon le mode opératoire de la préparation 2A, stade a), à partir du chlorure de *p*-chloro-benzyl-triphényl-phosphonium, on obtient avec un rendement de 94 % la N-t-butyloxycarbonyl-2-(p-chloro-cinnamyl)-pipéridine, sous forme d'une huile jaune.
- Stade b) La déprotection par l'acide trifluoroacétique est pratiquée selon le mode opératoire de la préparation 2A, stade b). Le produit obtenu sous forme d'une huile jaune est un mélange des isomères Z et E. Par chromato-flash sur silice, l'élution par le mélange dichlorométhane/méthanol à 10 % 90/10 v/v permet d'obtenir avec un rendement de 24 % la E-2-(*p*-chloro-cinnamyl)-pipéridine, sous forme d'une huile jaune pâle.

### Préparation 2D : E-2-(m-chloro-cinnamyl)-pipéridine (f. III ; Ar = p-chloro-phényle, n = 2)

- Stade a) Selon un mode opératoire similaire à celui de la préparation 2A, stade a), à partir du chlorure de *m*-chloro-benzyl-triphényl-phosphonium, on obtient avec un rendement de 99 % la N-t-butyloxycarbonyl-2-(*m*-chloro-cinnamyl)-pipéridine.
- Stade b) La déprotection par l'acide trifluoroacétique est pratiquée selon le mode opératoire de la préparation 2A, stade b). Le produit obtenu est un mélange des isomères Z et E. Par chromato-flash sur silice, l'élution par le mélange dichlorométhane/méthanol ammoniacal à 10 % 90/10 v/v permet d'obtenir l'isomère E dans les fractions les plus polaires. Les fractions intermédiaires sont soumises à une deuxième chromatographie dans les mêmes conditions. L'ensemble des fractions riches en isomère E sont réunies, et, comme il est décrit à la préparation 2B, stade b), on en fait le chlorhydrate qui est recristallisé dans l'isopropanol, puis traité en milieu alcalin, extrait à l'éther et concentré pour fournir la E-2-(*m*-chloro-cinnamyl)-pipéridine, avec un rendement de 24 %.

### Préparation 2E : E-2-(3,4-dichloro)-cinnamyl-pipéridine (f. III ; Ar = 3,4-dichloro-phényle, n = 2)

- Stade a) Selon le procédé 2A, stade a), à partir du chlorure de 3,4 dichloro-benzyl-triphényl-phosphonium, on obtient la N-*t*-butyloxycarbonyl-2-(3,4-dichloro)-cinnamyl-pipéridine sous forme d'une huile jaune. Rdt : 91 %.
   - - CCM :: Rf = 0,60- 0,80 (dichlorométhane)
- Stade b) La déprotection par l'acide trifluoro-acétique et la séparation de l'isomère E sont conduites selon le mode opératoire décrit au stade b) de la préparation 2D. On obtient avec un rendement de 18 % la E-2-(3,4-dichloro)-cinnamyl-pipéridine, sous forme d'une huile jaune.
   - - CCM :: Rf = 0,35- 0,55 (dichlorométhane/MeOH ammoniacal à 10% 90/10 v/v)

### Préparation 2F : E-2-(p-méthyl-cinnamyl)-pipéridine (f. III ; Ar = p-toluyle, n = 2)

- Stade a) Selon le procédé 2A, stade a), à partir du chlorure de *p*-méthylbenzyl-triphényl-phosphonium, on obtient la N-t-butyloxycarbonyl-2-(p-méthyl-cinnamyl)-pipéridine (f. V ; Ar = p-toluyle, n = 2) sous forme d'une huile jaune orangée. Rdt : 98 %
   - - CCM :: Rf = 0,25- 0,55 (dichlorométhane)
- Stade b) L'intermédiaire V du stade précédent est déprotégé et l'isomère E est séparé selon le mode opératoire de la préparation 2D, stade b). On obtient avec un rendement de 14 % la E-2-(*p*-méthyl-cinnamyl)-pipéridine, sous forme d'une huile jaune. Le chlorhydrate correspondant est un solide blanc dont le point de fusion vaut 178 °C (isopropanol).

### Préparation 2G : E-2-(p-trifluorométhyl-cinnamyl)-pipéridine (f. III ; Ar = p-trifluorométhylphényle, n = 2)

- Stade a) Selon le procédé 2A, stade a), à partir du chlorure de p-trifluorométhylbenzyl-triphényl-phosphonium, on obtient la N-Boc-2-(*p*-trifluorométhyl-cinnamyl)-pipéridine (f. V ; Ar = *p*-trifluorométhylphényle, n = 2) sous forme d'une huile jaune. Rdt : 94 %
- Stade b) L'intermédiaire V du stade précédent est déprotégé et l'isomère E est séparé selon le mode opératoire de la préparation 2B stade b) appliqué à l'isomère E. On obtient avec un rendement de 17 % la E-2-(*p*-trifluorométhyl-cinnamyl)-pipéridine.

### Préparation 2H : E-2-(p-méthoxy-cinnamyl)-pipéridine (f. III ; Ar = p-méthoxyphényle, n = 2)

- Stade a) Selon le procédé 2A, stade a), à partir du chlorure de *p*-méthoxybenzyl-triphényl-phosphonium, on obtient la N-Boc-2-(*p*-méthoxy-cinnamyl)-pipéridine (f. V ; Ar = *p*-méthoxyphényle, n = 2) sous forme d'une huile jaune qui est purifiée par chromatographie sur colonne de silice en éluant par un mélange de dichlorométhane/acétone 98/2 v/v. Rdt : 94 %.
   - - CCM :: Rf = 0,55- 0,75 (dichlorométhane/acétone 98/2 v/v)
- Stade b) L'intermédiaire V du stade précédent est déprotégé et l'isomère E est purifié selon le procédé 2D, stade b). On obtient avec un rendement de 15 % la E-2-(*p*-méthoxy-cinnamyl)-pipéridine sous forme d'une huile jaune.
   - - CCM :: Rf = 0,45- 0,70 (dichlorométhane/MeOH ammoniacal à 10 % 90/10 v/v).

### Préparation 2I : E-2-(1-napht-1-yl-propen-3-yl)-pipéridine (f. III ; Ar = napht-1-yle, n = 2)

- Stade a) Selon le procédé 2A, stade a), à partir du chlorure de napht-1-yl-méthyl-triphényl-phosphonium, on obtient la N-Boc-2-(1-napht-1-yl-propen-3-yl)-pipéridine (f. V ; Ar = napht-1-yle, n = 2) avec un rendement de 79 %.
- Stade b) L'intermédiaire V du stade précédent est déprotégé et l'isomère E est séparé selon un mode opératoire similaire à celui de la préparation 2B, stade b). On obtient avec un rendement de 19 % la E-2-(1-napht-1-yl-propen-3-yl)pipéridine.

### Préparation 2J : E-2-(1-thien-2-yl-propen-3-yl)-pipéridine (f. III ; Ar = thien-2-yle, n = 2)

- Stade a) Selon un mode opératoire similaire à celui de la préparation 2A, stade a), à partir du chlorure de thien-2-yl-méthyl-triphényl-phosphonium, on obtient avec un rendement de 91 % la N-Boc-2-(1-thien-2-yl-propen-3-yl)-pipéridine.
- Stade b) La déprotection est conduite selon le procédé 2B stade b). On obtient la E-2-(1-thien-2-yl-propen-3-yl)pipéridine avec un rendement de 5 % .

### Préparation 2K : Z-2-cinnamyl-pyrrolidine (f. III-Z ; Ar = C₆H₅, n = 1)

- Stade a) Dans un réacteur protégé de l'humidité, on introduit 235 ml d'éthanol absolu puis, sous agitation, 2,16 g (0,094 mol) de sodium décapé. Après dissolution, à 20- 25 °C, on ajoute 36,5 g (0,094 mol) de chlorure de benzyl-triphénylphosphonium. La solution jaune est maintenue sous agitation 30 min puis on introduit en 2 min environ une solution de 20 g (0,094 mol) de l'acétaldéhyde (f. VI ; n = 1) obtenu précédemment, dans 47 ml d'éthanol absolu. La suspension blanche est maintenue sous agitation 45 min à 20- 25 °C, puis l'insoluble est filtré sur buchner et écarté. Le filtrat est évaporé sous vide et sur bain-marie. Le résidu huileux, repris dans 300 ml de pentane, est maintenu 2 h à 0 °C sous agitation, puis filtré, et concentré. Cette dernière opération est répétée. On obtient 24,5 g (Rdt = 91 %) de N-Boc-2-cinnamyl-pyrrolidine brute (f.V ; Ar = C₆H₅, n = 1), sous forme d'une huile jaune, qui est engagée telle quelle au stade suivant.
- Stade b) L'intermédiaire V du stade précédent est déprotégé et l'isomère Z est séparé selon un mode opératoire similaire à celui de la préparation 2D, stade b). On obtient avec un rendement de 28 % la Z-2-cinnamyl-pyrrolidine.
   - - CCM :: Rf = 0,35- 0,50 (dichlorométhane/MeOH ammoniacal à 10 % 90/10 v/v).

### Préparation 2L : E-2-(p-fluoro-cinnamyl)-pyrrolidine (f. III ; Ar = p-fluoro-phényle, n = 1)

- Stade a) Dans un réacteur protégé de l'humidité, on introduit 42 g (0,103 mol) de chlorure de p-fluorobenzyl-triphénylphosphonium dans 235 ml de toluène déshydraté sur tamis moléculaire. On introduit ensuite en 10 min 60 ml d'une solution 2,5 M de n-butyl-lithium dans l'hexane. La suspension rouge est maintenue sous agitation 2 h à 20- 25 °C puis on introduit en 5 min une solution de 20 g (0,094 mol) de l'acétaldéhyde (f. VI ; n = 1) obtenu précédemment, dans 42,2 ml de toluène. La suspension rouge foncé obtenue est maintenue 16 h sous agitation, puis on introduit 80 ml d'une solution saturée de chlorure d'ammonium en refroidissant à 20 °C. Après agitation 15 min, l'insoluble est filtré et écarté. La phase toluénique du filtrat est décantée, séchée sur Na₂SO₄ et évaporée. Le résidu huileux marron est concrétisé dans 300 ml de n-pentane, et le nouvel insoluble est filtré et éliminé ; le filtrat est concentré. On répète cette dernière étape de purification. On obtient 23,6 g (Rdt : 82 %) de N-Boc-2-(*p*-fluoro-cinnamyl)-pyrrolidine brute (f. V ; Ar = *p*-fluorophényle, n= 1) sous forme d'une huile orange.
- Stade b) La déprotection par l'acide trifluoroacétique et la séparation de l'isomère E sont conduites selon le mode opératoire de la préparation 2B, stade b) appliqué à l'isomère E, en recristallisant dans l'acétate d'éthyle. On obtient le chlorhydrate de E-2-(*p*-fluoro-cinnamyl)-pyrrolidine, sous forme d'un précipité blanc. Le retour à la base fournit la E-2-(*p*-fluoro-cinnamyl)-pyrrolidine avec un rendement de 19 %.
   - - CCM :: Rf = 0,30- 0,50 (dichlorométhane/MeOH ammoniacal à 10 % 90/10 v/v).

### Exemple 1.1 : (+/-)-E-2-cinnamyl-1-cyclopropylméthylpyrrolidine (f. I ; Ar = C₆H₅, m = 1, n = 1, R = cyclopropyle)

- Stade a) Dans un réacteur protégé de l'humidité, on introduit 4,5 g (0,024 mol) de E-2-cinnamyl-pyrrolidine (préparation 1, stade b) en solution dans 100 ml de dichlorométhane déshydraté sur tamis moléculaire. Sous agitation on ajoute 2,5 g (3,5 ml - 0,025 mol) de triéthylamine puis, à une température inférieure à 10 °C en 10 min environ, 2,5 g (2,2 ml - 0,024 mol) de chlorure d'acide cyclopropane carboxylique (f. IV ; m = 1, R = cyclopropyle). La solution marron est maintenue sous agitation 1 h et le mélange est extrait successivement par :
   - 30 ml de solution d'ammoniaque à 10 % puis 30 ml d'eau,
   - 30 ml de solution HCl à 10 % puis 30 ml d'eau,
   - 30 ml de solution saturée en NaHCO₃ puis 30 ml d'eau.

   La phase organique est déshydratée sur Na₂SO₄ puis le solvant évaporé sous vide et sur bain-marie. Le résidu huileux (5,9 g) est purifié par chromatographie sur colonne de silice. L'élution par le mélange dichlorométhane/acétone 95/5 v/v permet d'obtenir la 2-cinnamyl-1-cyclopropane-carbonyl-pyrrolidine (f. II ; Ar = C₆H₅, m = 1, n = 1, R = cyclopropyle) pure, sous forme d'une huile jaune.
   - Poids :: 4,2 g Rdt : 65,8 %
   - - CCM :: Rf = 0,35- 0,45 (dichlorométhane/acétone 95/5 v/v)
   - - RMN :: 0,60- 1,20 (m, 4H) ; 1,40- 2,05 (m, 5H) ; 2,10- 2,90 (m, 2H) ; 3,40- 3,75 (m, 2H) ; 4,00-4,35 (m, 1H) ; 5,85- 6,60 (m, 2H) ; 7,10- 7,50 (m, 5H).
- Stade b) Sous atmosphère d'azote, à l'abri de l'humidité et sans dépasser 0 °C on prépare d'une part une suspension de 1,9 g (0,049 mol) d'hydrure de lithium aluminium (LAH) dans 25 ml de THF déshydraté sur tamis moléculaire, et d'autre part une solution de 2,15 g (0,016 mol) de chlorure d'aluminium dans 25 ml d'éther diéthylique également déshydraté sur tamis. Après 30 min de contact pour chaque préparation, la suspension LAH/THF est introduite en 10 min à 0 °C dans la solution éthérée de AlCl₃, puis on introduit à cette température et en 10 min une solution de 4,0 g (0,016 mol) de l'amide (II) obtenu au stade précédent dans 16 ml de THF anhydre. Après 30 min à 0 °C le mélange est porté 10 min au reflux puis refroidi rapidement à 0 °C. On ajoute alors goutte à goutte avec précautions 2,9 ml de solution NaOH 15 % (p/v) puis 3,6 ml d'eau. Après 30 min de contact le mélange est filtré sur buchner garni par un lit de terre d'infusoires. Le filtrat est concentré sous vide et sur bain-marie pour obtenir la E-2-cinnamyl-N-cyclopropylméthylpyrrolidine, qui est constatée pure par CCM.
   - Poids :: 3,2 g Rdt : 84,6 %
   - - CCM :: Rf = 0,55- 0,75 (dichlorométhane/acétone 95/5 v/v)
   - - RMN :: 0,00- 0,30 (m, 2H) ; 0,40- 0,70 (m, 2H) ; 0,80- 1,20 (m, 1H) ; 1,40- 3,00 (m, 10H) ; 3,20-3,50 (m, 1H) ; 6,00- 6,60 (m, 2H) ; 7,10- 7,55 (m, 5H).

**Chlorhydrate** : La base est dissoute dans 50 ml de dichlorométhane, on ajoute 5 ml d'éther chlorhydrique 5 N, puis élimine les solvants par distillation. Le résidu solide est cristallisé par dissolution dans 50 ml d'acétate d'éthyle. L'insoluble blanc est filtré, séché sous vide jusqu'à poids constant. F = 163 °C.
- Analyse (C₁₇ H₂₄ Cl N) : % C, H, Cl, N conformes.
- IR (KBr) : 2950, 2500, 1460, 1440, 1050, 1020, 970, 940, 830, 740, 690 cm⁻¹.

### Exemple 1.2 : (+)-E-2-cinnamyl-1-cyclopropylméthylpyrrolidine (f. I ; Ar = C₆H₅, m = 1, n = 1, R = cyclopropyle)

Le composé est préparé tel que décrit à l'exemple 1.1 précédent à partir de (-)-E-2-cinnamyl-pyrrolidine (préparation 1, stade c).
- Stade a) (+)-E-2-cinnamyl-1-cyclopropane-carbonyl-pyrrolidine (f. II ; Ar = C₆H₅, m = 1, n = 1, R = cyclopropyle)
   Rdt : 100 % [α]_{D} = 24,5 ° (c = 1, dichlorométhane)
   - - CCM :: Rf = 0,35- 0,50 (dichlorométhane/acétone 95/5 v/v)
- Stade b) (+)-E-2-cinnamyl-1-cyclopropylméthyl-pyrrolidine Rdt : 96 % [α]_{D} = + 91,9 ° (c = 1, dichlorométhane)
   - - CCM :: Rf = 0,45- 0,60 (dichlorométhane/MeOH ammoniacal à 10% 95/5 v/v)
   - - RMN :: Identique au produit racémique (Ex. 1.1).

**Chlorhydrate** : F = 196- 198 °C (isopropanol).
- Analyse (C₁₇ H₂₁ Cl N) : % C, H, N conformes.
- IR (KBr) : Identique au produit racémique (Ex. 1.1).

### Exemple 1.3 : (-)-E-2-cinnamyl-1-cyclopropylméthylpyrrolidine (f. I ; Ar = C₆H₅, m = 1, n = 1, R = cyclopropyle)

Le composé est préparé tel que décrit à l'exemple 1.1 précédent à partir de (+)-E-2-cinnamyl-pyrrolidine (préparation 1, stade d).
- Stade a) (-)-E-2-cinnamyl-1-cyclopropane-carbonyl-pyrrolidine (f. II ; Ar = C₆H₅, m = 1, n = 1, R = cyclopropyle)
   Rdt : 100 % [α]_{D} = - 20,8 ° (c = 1, dichlorométhane)
   - CCM : Rf = 0,35- 0,50 (dichlorométhane/acétone 95/5 v/v)
- Stade b) (-)-E-2-cinnamyl-1-cyclopropylméthyl-pyrrolidine Rdt : 94 % [α]_{D} = + 91,8 ° (c = 1, dichlorométhane)
   - - CCM :: Rf = 0,45- 0,60 (dichlorométhane/MeOH ammoniacal à 10 % 95/5 v/v)
   - - RMN :: Identique au produit racémique (Ex. 1.1).

**Chlorhydrate** : F = 196- 198 °C (isopropanol).
- Analyse (C₁₇ H₂₄ Cl N) : % C, H, N conformes.
- IR : Identique au produit racémique (Ex. 1.1).

### Exemple 1.4 : E-2-cinnamyl-1-cyclobutylméthylpyrrolidine (f. I ; Ar = C₆H₅, m = 1, n = 1, R = cyclobutyle)

- Stade a) Selon le mode opératoire de l'exemple 1.1, stade a) à partir de 2-cinnamyl-pyrrolidine et de chlorure d'acide cyclobutane carboxylique, on obtient avec un rendement de 95 % la 2-cinnamyl-1-cyclobutane-carbonylpyrrolidine (f. II ; Ar = C₆H₅, m = 1, n = 1, R = cyclobutyle) que l'on engage telle quelle dans le stade suivant.
- Stade b) La réduction pratiquée selon le mode opératoire de l'exemple 1.1, stade b) avec LAH - AlCl₃ conduit à la 2-cinnamyl-1-cyclobutylméthylpyrrolidine avec un rendement de 45 %.
   - - CCM :: Rf = 0,50- 0,60 (dichlorométhane/MeOH ammoniacal à 10 % 95/5 v/v)
   - - RMN :: 1,40- 3,20 (m, 18H) ; 5,95- 6,95 (m, 2H) ; 7,00- 7,45 (m, 5H).

**Chlorhydrate** : Préparé comme décrit à l'exemple 1.1, le produit est cristallisé dans le mélange dichlorométhane/éther éthylique. F = 170- 171 °C.
- Analyse (C₁₈ H₂₆ Cl N) : % C, H, Cl, N conformes.
- IR (KBr) : 2950, 2500, 1440, 1240, 1020, 970, 740, 680 cm⁻¹.

### Exemple 1.5 : E-2-cinnamyl-1-cyclopropyléthyl-pyrrolidine (f. I ; Ar = C₆H₅, m = 2, n = 1, R = cyclopropyle)

- Stade a) Dans un réacteur protégé de l'humidité, on introduit 1 g (0,010 mol) d'acide cyclopropane acétique, 2,34 g (0,012 mol) de E-2-cinnamyl-pyrrolidine en solution dans 80 ml de dichlorométhane déshydraté sur tamis moléculaire et 2,9 g (0,015 mol) de chlorhydrate de 1-(3-diméthylaminopropyl)-3-éthyl-carbodiimide. La solution marron est maintenue sous agitation 16 h à 20- 25 °C et le mélange est extrait successivement par :
   - 50 ml de solution HCl 1 N, puis 2 x 50 ml d'eau,
   - 50 ml de solution saturée en NaHCO₃, puis 2 x 50 ml d'eau. La phase organique est déshydratée sur Na₂SO₄, filtrée, puis le solvant est évaporé sous vide et sur bain-marie. On obtient la 2-cinnamyl-1-cyclopropane-acétyl-pyrrolidine (f. Il ; Ar = C₆H₅, m = 2, n = 1, R = cyclopropyle) sous forme d'une huile marron. Poids = 2,2 g Rdt : 82 %
   - CCM : Rf = 0,85- 0,95 (dichlorométhane/MeOH ammoniacal à 10 % 92/8 v/v)
- Stade b) L'amide II du stade précédent est réduit selon le mode opératoire décrit à l'exemple 1.1, stade b), suivi par une étape de purification par chromatographie sur colonne de silice. L'élution par le mélange dichlorométhane/méthanol ammoniacal à 10 % 90/10 v/v permet d'obtenir la E-2-cinnamyl-1-cyclopropyléthyl-pyrrolidine, sous forme d'une huile jaune.
   - Poids :: 1,7 g Rdt : 81 %
   - - CCM :: Rf = 0,85 - 1,00 (dichlorométhane/MeOH ammoniacal à 10 % 90/10 v/v)
   - - RMN :: 0- 0,10 (m,2H) ; 0,40- 0,50 (m,2H) ; 0,70- 0,80 (m,lH); 1,30- 1,90 (m,7H) ; 2,10 - 2,30 (m,2H) ; 2,30-2,40 (m,1H) ; 2,50- 2,60 (m,1H) ; 2,90- 3,00 (m,1H) ; 3,10- 3,20 (m,1H) ; 6,10- 6,20 (m,1H) 6,45 (d,1H) ; 7,10-7,40 (m,5H)

**Chlorhydrate** : F = 188 °C (isopropanol).
- Analyse (C₁₈ H₂₆ Cl N) : % C, H, Cl, N conformes.
- IR : 2900, 2400, 1420, 1020, 960, 900, 740, 700 cm⁻¹.

### Exemple 1.6 : E-2-cinnamyl-1-phénéthyl-pyrrolidine (f. I ; Ar = C₆H₅, m = 2, n = 1, R = C₆H₅)

- Stade a) On pratique comme à l'exemple 1.1, stade a) à partir de E-2-cinnamyl-pyrrolidine et de chlorure d'acide phényl-acétique. On obtient avec un rendement de 97 % la E-2-cinnamyl-1-phénylacétyl-pyrrolidine que l'on engage telle quelle dans le stade suivant.
- Stade b) La réduction pratiquée selon le mode opératoire de l'exemple 1.5, stade b) conduit à la E-2-cinnamyl-1-phénétylpyrrolidine, avec un rendement de 45 %.
   - - CCM :: Rf = 0,80- 0,95 (dichlorométhane/MeOH ammoniacal à 10 % 90/10 v/v)
   - - RMN :: 1,50- 2,00 (m,4H) ; 2,10- 2,30 (m,2H) ; 2,30- 2,60 (m,3H) ; 2,70- 2,90 (m,2H) ; 3,00- 3,20 (m,1H) ; 3,20- 3,30 (m,1H) ; 6,10- 6,20 (m,1H) ; 6,45 (d,1H) ; 7,10- 7,40 (m,10H)

**Chlorhydrate** : F = 120- 122 °C (acétate d'éthyle/éther éthylique).
- Analyse (C₂₁ H₂₆ Cl N) : % C, H, Cl, N conformes.
- IR (KBr) : 2900 ; 2450 ; 1600 ; 1490 ; 1450 ; 1050 ; 990 ; 740 ; 690 cm⁻¹.

### Exemple 2A.1 : E-2-cinnamyl-1-cyclopropylméthylpipéridine (f. I ; Ar = C₆H₅, m = 1, n = 2, R = cyclopropyle)

- Stade a) On pratique comme à l'exemple 1.1, stade a) à partir de E-2-cinnamyl-pipéridine (préparation 2A, stade b) et de chlorure d'acide cyclopropane carboxylique, pour obtenir la E-2-cinnamyl-1-cyclopropane-carbonyl-pipéridine (f. II ; Ar = C₆H₅, m = 1, n = 2, R = cyclopropyle). Rdt : 95 %.
   - - CCM :: Rf = 0,80- 0,90 (acétate d'éthyle)
   - - RMN :: 0,50- 1,10 (m, 5H) ; 1,30- 1,90 (m, 8H) ; 4,00- 5,00 (m, 1H) ; 5,90- 6,60 (m, 2H) ; 7,10-7,40 (m, 5H).
- Stade b) L'amide (II) du stade précédent est réduit comme décrit à l'exemple 1.1, stade b) pour obtenir la E-2-cinnamyl-1-cycloproprane-carbonyl-pipéridine. Rdt : 86 %
   - - CCM :: Rf = 0,65- 0,80 (dichlorométhane/MeOH ammoniacal à 10 % 95/5 v/v)
   - - RMN :: 0,10- 0,20 (m, 2H) ; 0,20- 0,60 (m, 2H) ; 0,60- 1,00 (m, 1H) ; 1,10- 1,80 (m, 6H) ; 2,10- 2,80 (m, 6H) ; 2,60- 3,20 (m, 1H) ; 5,95- 6,55 (m, 2H) ; 7,10- 7,40 (m, 5H).

**Chlorhydrate** : Le produit est préparé dans les conditions décrites à l'exemple 1.1, la cristallisation étant effectuée dans l'acétate d'éthyle à ébullition. F = 152 °C.
- Analyse (C₁₈ H₂₆ Cl N) : % C, H, Cl, N conformes ;
- IR (KBr) :2950, 2680, 2500, 1440, 1360, 1260, 1200, 1120, 1020, 980, 950, 820, 800, 770, 740, 680 cm⁻¹.

### Exemple 2A.2 : (+) -E-2-cinnamyl-1-cyclopropylméthyl-pipéridine (f. I ; Ar = C₆H₅, m = 1, n = 2, R = cyclopropyle)

Le composé est préparé tel que décrit à l'exemple 2A.1 précédent à partir de (-)-E-2-cinnamyl-pipéridine (préparation 2A, stade c).
- Stade a (-)-E-2-cinnamyl-1-cyclopropane-carbonyl-pipéridine (f. II ; Ar = C₆H₅, m = 1, n = 2, R = cyclopropyle)
   Rdt : 100 % [α]_{D} = -6,4 ° (c = 4, dichlorométhane)
   - - CCM :: Rf = 0,60- 0,75 (dichlorométhane/acétone 95/5 v/v)
- Stade b) (+)-E-2-cinnamyl-1-cyclopropylméthyl-pipéridine Rdt : 95 % [α]_{D} = + 27,7 ° (c = 1, dichlorométhane)
   - - CCM :: 0,60- 0,75 (dichlorométhane/acétone 95/5 v/v)
   - - RMN :: Identique au produit racémique (Ex. 2A.1)

**Chlorhydrate** : F = 153 °C (acétate d'éthyle).
- Analyse (C₁₈ H₂₆ Cl N) : % C, H, Cl, N conformes ;
- IR (KBr) : Identique au produit racémique (Ex. 2A.1)

### Exemple 2A.3 : (-1-E-2-cinnamyl-cyclopropylméthyl-pipéridine (f. I ; Ar = C₆H₅, m = 1, n = 2, R = cyclopropyle)

Le composé est préparé tel que décrit à l'exemple 2A.1 précédent à partir de (+)-E-2-cinnamyl-pipéridine (préparation 2A, stade d).
- Stade a) (+)-E-2-cinnamyl-1-cyclopropane-carbonyl-pipéridine (f. II ; Ar = C₆H₅, m = 1, n = 2, R = cyclopropyle)
   Rdt : 100 % [α]_{D} = + 6,3 ° (c = 4,5, dichlorométhane)
   - - CCM :: Rf = 0,60- 0,75 (dichlorométhane/acétone 95/5 v/v)
- Stade b) (-)-E-2-cinnamyl-1-cyclopropylméthyl-pipéridine Rdt : 94 % [α]_{D} = - 28,5 ° (c = 1, dichlorométhane)
   - - CCM :: Rf = 0,45- 0,65 (dichlorométhane/MeOH ammoniacal à 10 % 95/5 v/v)
   - - RMN :: Identique au produit racémique (Ex.2A.1).

**Chlorhydrate** : F = 151- 152 °C (éther éthylique/isopropanol).
- Analyse (C₁₈ H₂₆ Cl N) : % C, H, Cl, N conformes.
- IR : Identique au produit racémique (Ex.2A.1).

### Exemple 2A.4 : E-2-cinnamyl-1-cyclobutylméthylpipéridine (f. I ; Ar = C₆H₅, m = 1, n = 2, R = cyclobutyle)

- Stade a) On procède comme à l'exemple 1.1 stade a) à partir de E-2-cinnamyl-pipéridine et de chlorure d'acide cyclobutane carboxylique, pour obtenir la E-2-cinnamyl-1-cyclobutane-carbonyl-pipéridine (f. II ; Ar = C₆H₅, m = 1, n = 2, R = cyclobutyle). Rdt : 99 %.
   - - CCM :: Rf = 0,40- 0,55 (dichlorométhane/acétone 95/5 v/v)
- Stade b) L'intermédiaire du stade précédent est réduit dans les conditions décrites à l'exemple 1.1, stade b) pour obtenir la E-2-cinnamyl-1-cyclobutane-carbonyl-pipéridine. Rdt : 86 %.
   - - CCM :: Rf = 0,65- 0,90 (dichlorométhane/MeOH ammoniacal à 10 % 95/5 v/v)
   - - RMN :: 1,20- 2,60 (m, 18H) ; 2,70- 2,80 (m, 2H) ; 6,10- 6,20 (m, 1H) ; 6,40 (d, 1H) ; 7,10- 7,40 (m, 5H).

**Chlorhydrate** : F = 163 °C (acétate d'éthyle).
- Analyse (C₁₉ H₂₈ Cl N) : % C, H, Cl, N conformes ;
- IR (KBr) : 2900, 2500, 1440, 1210, 1080, 980, 860, 700 cm⁻¹.

### Exemple 2A.5 : E-2-cinnamyl-1-phénéthyl-pipéridine (f. I ; Ar = C₆H₅, m = 2, n = 2, R = C₆H₅)

Le composé est préparé tel que décrit à l'exemple 1.6 précédent à partir de E-2-cinnamyl-pipéridine et de chlorure d'acide phényl-acétique.
- Stade a) E-2-cinnamyl-1-phényl-acétyl-pipéridine (f. II ; Ar = C₆H₅, m = 2, n = 2, R = C₆H₅)
   Rdt : 99 %
   - - CCM :: Rf = 0,50- 0,60 (dichlorométhane/acétone 95/5 v/v)
- Stade b) E-2-cinnamyl-1-phénétyl-pipéridine
   Rdt : 73 %
   - - CCM :: Rf = 0,60- 0,80 (dichlorométhane/MeOH ammoniacal à 10 % 98/2 v/v)
   - - RMN :: 1,30- 1,80 (m, 6H) ; 2,30- 2,60 (m, 4H) ; 2,80- 3,00 (m, 5H) ; 6,10- 6,20 (m, 1H) ; 6,40 (d, 1H) ; 7,10-7,40 (m, 10H)

### Chlorhydrate : Produit hygroscopique F = 95- 100 °C (éther éthylique/isopropanol)

- Analyse (C₂₂ H₂₈ Cl N) : % C, H, Cl, N conformes ;
- IR (KBr) : 3400, 2900, 2500, 1600, 1430, 1260, 1080, 740, 690 cm⁻¹.

### Exemple 2A.6 : Z-2-cinnamyl-1-cyclopropylméthylpipéridine (f. I ; Ar = C₆H₅, m = 1, n = 2, R = cyclopropyle)

Le composé est préparé tel que décrit à l'exemple 2A.1 précédent à partir de Z-2-cinnamyl-pipéridine (préparation 2A, stade b).
- Stade a) z-2-cinnamyl-l-cyclopropane-carbonyl-pipéridine (f. II ; Ar = C₆H₅, m = 1, n = 2, R = cyclopropyle)
   Rdt : 96 %
   - - CCM :: Rf = 0,75- 0,80 (acétate d'éthyle)
   - - RMN :: 0,60- 0,85 (m, 2H) ; 0,85- 1,10 (m, 2H) ; 1,10- 1,90 (m, 9H) ; 2,40- 3,00 (m, 2H) ; 3,80- 5,00 (m, 1H) ; 5,45- 5,85 (m, 1H) ; 6,40- 6,70 (m, 1H) ; 7,10- 7,50 (m, 5H).
- Stade b Z-2-cinnamyl-1-cyclopropylméthylpipéridine. Rdt : 91,2 %
   - - CCM :: Rf = 0,50- 0,70 (dichlorométhane/MeOH ammoniacal à 10 % 95/5 v/v)
   - - RMN :: 0,00- 0,10 (m, 2H) ; 0,30- 0,60 (m, 2H) ; 0,60- 1,00 (m, 1H) ; 1,10- 1,80 (m, 6H) ; 2,00- 2,65 (m, 6H) ; 2,90- 3,20 (m, 1H) ; 5,45- 5,90 (m, 1H) ; 6,35- 6,60 (m, 1H) ; 7,10- 7,50 (m, 5H).

Chlorhydrate : F = 112 °C (éther éthylique).
- Analyse (C₁₈ H₂₆ Cl N) : % C, H, Cl, N conformes ;
- IR (KBr) : 3400, 2800, 2600, 2500, 1440, 1200, 1020, 1000, 960, 800, 770, 680 cm⁻¹.

### Exemple 28.1 : E-2-(p-fluoro-cinnamyl)-1-cyclopropylméthyl-pyrrolidine (f. I-E ; Ar = p-fluoro-phényle, m = 1, n = 2, R = cyclopropyle)

Dans un réacteur protégé de l'humidité, on introduit 2,3 g (0,010 mol) de E-2-(p-fluoro-cinnamyl)-pipéridine (préparation 2B) en solution dans 25,2 ml d'acétonitrile, puis en 2 min 1,56 g (1,1 ml - 0,012 mol) de bromométhylcyclopropane. Le milieu est maintenu sous agitation à température ambiante pendant 1 h 30 min, porté à 60° C pendant 4 h, puis ramené à 20 ± 3° C pendant 16 h. Le solvant est évaporé sous vide et sur bain-marie. Le résidu huileux est repris dans de l'eau, acidifié et extrait à l'éther. Après décantation, la phase aqueuse refroidie est alcalinisée par la soude, extraite à l'éther et lavée par une solution saturée de NaCl. La phase organique est déshydratée sur Na₂SO₄, puis le solvant éliminé par distillation. Le résidu est purifié par chromato-flash en éluant par le mélange dichlorométhane/méthanol ammoniacal à 10 % 90/10 v/v, et soumis à un second traitement acide-alcalin. La E-2-(*p*-fluoro-cinnamyl)-1-cyclopropylméthyl-pyrrolidine est obtenue sous forme d'une huile jaune.
- Poids :: 0,9 g Rdt : 31 %
- - CCM :: Rf = 0,35- 0,60 (dichlorométhane/MeOH ammoniacal à 10 % 90/10 v/v)
- - RMN :: 0,00- 0,20 (m, 2H) ; 0,40- 0,60 (m, 2H) ; 0,70- 1,00 (m, 1H) ; 1,10- 1,90 (m, 6H) - 2,00- 2,70 (m, 6H) ; 2,8-3,20 (m, 1H) ; 5,90- 6,50 (m, 2H) ; 6,80- 7,40 (m, 4H).

**Chlorhydrate** : F = 135 °C (acétate d'éthyle).
- Analyse (C₁₈ H₂₅ Cl F N) : % C, H, Cl, F, N conformes ;
- IR (KBr) : 3500, 2850, 2450, 1590, 1500, 1450, 1220, 1000, 860, 520 cm⁻¹.

### Exemple 2B.2 : Z-2-(p-fluoro-cinnamyl)-1-cyclopropylméthyl-pipéridine (f. I- Z ; Ar = p-fluoro-phényle, m = 1, n = 2, R = cyclopropyle)

Le composé est préparé tel que décrit à l'exemple 2B.1 précédent à partir de Z-2-(*p*-fluoro-cinnamyl)-pipéridine (préparation 2B) avec un rendement de 43 %.
- - CCM :: Rf = 0,35- 0,60 (dichlorométhane/MeOH ammoniacal à 10 % 90/10 v/v)
- - RMN :: 0,00- 0,10 (m, 2H) ; 0,20- 0,60 (m, 2H) ; 0,60- 1,00 (m, 1H) ; 1,10- 1,80 (m, 6H) ; 2,00- 2,60 (m, 6H) ; 2,80- 3,20 (m, 1H) ; 5,50- 5,90 (m, 1H) ; 6,30- 6,60 (m, 1H) ; 6,90- 7,30 (m,4H).

**Chlorhydrate** : F = 102 °C (acétate d'éthyle).
- Analyse (C₁₈ H₂₅ Cl F N) : % C, H, Cl, F, N conformes ;
- IR (KBr) : 3400, 2900, 2400, 1600, 1500, 1440, 1220, 1160, 1000, 1090, 1030, 860, 840, 750, 620, 520 cm⁻¹.

### Exemple 2C : E-2-(p-chloro-cinnamyl)-1-cyclopropylméthylpipéridine (f. I ; Ar = p-chloro-phényle, m = 1, n = 2, R = cyclopropyle)

Le composé est préparé tel que décrit à l'exemple 2B.1 précédent à partir de E-2-(*p*-chloro-cinnamyl)-pipéridine (préparation 2C avec un rendement de 44 %.
- - RMN :: 0,00- 0,20 (m, 2H) ; 0,40- 0,60 (m, 2H) ; 0,60- 1,00 (m, 1H) ; 1,10- 1,80 (m, 6H) ; 2,10- 2,80 (m, 6H) ; 2,90- 3,20 (m, 1H) ; 5,90- 6,70 (m, 2H) ; 7,30 (m, 4H).

**Chlorhydrate** : F = 173 °C (acétate d'éthyle).
- Analyse (C₁₈ H₂₅ Cl₂ N) : % C, H, Cl, N conformes ;
- IR (KBr) : 3500, 2900, 2500, 1440, 1080, 970, 950, 850 cm⁻¹.

### Exemple 2D : E-2-(m-chloro-cinnamyl)-1-cyclopropylméthyl pipéridine (f. I ; Ar = m-chloro-phényle, m = 1, n = 2, R = cyclopropyle)

Le composé est préparé tel que décrit à l'exemple 1.1 à partir de E-2-(m-chloro-cinnamyl)-pipéridine (préparation 2D).
- Stade a E-2-(*m*-chloro-cinnamyl)-1-cyclopropane-carbonyl-pipéridine (f. II ; Ar = *m*-chloro-phényle, m = 1, n = 2, R = cyclopropyle). Rdt : 91 %.
- Stade b) E-2(*m*-chloro-cinnamyl)-1-cyclopropylméthyl-pipéridine. Rdt : 95 %.
   - - CMM :: Rf = 0,7 (dichlorométhane/MeOH ammoniacal à 10 % 90/10 v/v).
   - - RMN :: 0,00- 0,30 (m, 2H) ; 0,60- 0,70 (m, 2H) ; 0,90- 1,10 (m, 1H) ; 1,30- 1,90 (m, 6H) ; 2,30- 2,80 (m, 6H) ; 3,10- 3,20 (m, 1H) ; 6,30- 6,50 (m, 2H) ; 7,20- 7,50 (m, 4H).

**Chlorhydrate** : F = 129 °C (acétate d'éthyle).
- Analyse (C₁₈ H₂₅ Cl₂ N) : % C, H, Cl, N conformes ;
- IR (KBr) : 3400, 2950, 2500, 1590, 1560, 1440, 1210,970, 780, 680, 560 cm⁻¹.

### Exemple 2E : E-2-(3,4-dichloro-cinnamyl)-1-cyclopropylméthyl-pipéridine (f. I ; Ar = 3,4-dichloro-phényle, m = 1, n = 2, R = cyclopropyle)

Le composé est préparé tel que décrit à l'exemple 1.1 à partir de E-2-(3,4-dichloro-cinnamyl)-pipéridine (préparation 2E).
- Stade a E-2-(3,4-dichloro-cinnamyl)-1-cyclopropane-carbonyl-pipéridine (f. II ; Ar = 3,4-dichlorophényle, m = 1, n = 2, R = cyclopropyle). Rdt : 91 %.
   - - CMM :: Rf = 0,90- 1,00 (dichlorométhane/MeOH ammoniacal à 10 % 90/10 v/v).
- Stade b) E-2-(3,4-dichloro-cinnamyl)-1-cyclopropylméthyl-pipéridine. Rdt : 79 %.
   - - CMM :: Rf = 0,65- 0,80 (dichlorométhane/MeOH ammoniacal à 10 % 90/10 v/v).
   - - RMN :: 0,00- 0,20 (m, 2H) ; 0,40- 0,70 (m, 2H) ; 0,70- 1,10 (m, 1H) ; 1,10- 1,80 (m, 6H) ; 2,10- 2,80 (m, 6H) ; 2,90- 3,20 (m, 1H) ; 6,20- 6,40 (m, 2H) ; 7,10- 7,50 (m, 3H).

**Chlorhydrate** : F = 153 °C (acétate d'éthyle).
- Analyse (C₁₈ H₂₄ Cl₃ N) : % C, H, Cl, N conformes ;
- IR (KBr) : 3400, 2900, 2500, 1450, 1130, 1020, 990, 820, 800 cm⁻¹.

### Exemple 2F : E-2-(p-méthyl-cinnamyl)-1-cyclopropylméthyl-pipéridine (f. I ; Ar = p-toluyle, m = 1, n = 2, R = cyclopropyle)

Le composé est préparé tel que décrit à l'exemple 1.1 à partir de E-2-(p-méthyl-cinnamyl)-pipéridine (préparation 2F).
- Stade a) E-2-(p-méthyl-cinnamyl)-1-cyclopropane-carbonyl-pipéridine (f. II ; Ar = p-toluyle, m = 1, n = 2,
   R = cyclopropyle). Rdt : 100 %.
- Stade b) E-2-(*p*-méthyl-cinnamyl)-1-cyclopropylméthyl-pipéridine. Rdt : 98 %.
   - - RMN :: 0,00- 0,20 (m, 2H) ; 0,40- 0,60 (m, 2H) ; 0,70- 1,10 (m, 1H) ; 1,10- 1,90 (m, 6H) ; 2,35 (s, 3H) ; 2,10-2,80 (m, 6H) ; 2,80- 3,20 (m, 1H) ; 5,90- 6,50 (m, 2H) ; 6,90- 7,30 (m,4H).

**Chlorhydrate** : F = 152 °C (acétate d'éthyle).
- Analyse (C₁₉ H₂₈ Cl N) : % C, H, Cl, N conformes ;
- IR (KBr) : 3300, 2900, 2500, 1500, 1450, 1420, 1250, 1020, 960, 800, 490 cm⁻¹.

### Exemple 2G : E-2-(p-trifluorométhyl-cinnamyl)-1-cyclopropyl-méthyl-pipéridine (f. I ; Ar = p-trifluorométhyl-phényle, m = 1, n = 2, R = cyclopropyle)

Le composé est préparé tel que décrit à l'exemple 1.1 à partir de E-2-(p-trifluorométhyl-cinnamyl)-pipéridine (préparation 2G).
- Stade a) E-2-(p-trifluorométhyl-cinnamyl)-1-cyclopropane-carbonyl-pipéridine (f. II ; Ar = p-trifluorométhyl-phényle, m = 1, n = 2, R = cyclopropyle). Rdt : 98 %.
- Stade b) E-2-(*p*-trifluorométhyl-cinnamyl)-1-cyclopropyl-méthyl-pipéridine. Rdt : 92 %.
   - - CCM :: Rf = 0,6 (dichlorométhane/MeOH ammoniacal à 10 % 90/10 v/v)
   - - RMN :: 0,00- 0,20 (m, 2H) ; 0,40- 0,60 (m, 2H) ; 0,80- 1,00 (m, 1H) ; 1,20- 1,80 (m, 6H) ; 2,30- 2,70 (m, 6H) ; 3,00- 3,10 (m, 1H) ; 6,20- 6,40 (m, 1H) ; 6,45 (d, 1H) ; 7,30- 7,70 (m,4H).

**Chlorhydrate** : F = 125 °C (acétate d'éthyle).
- Analyse (C₁₉ H₂₅ F₃ Cl N) : % C, H, F, Cl, N conformes ;
- IR (KBr) : 2950, 2450, 1430, 1320, 1160, 1120, 1070, 960, 790, 690 cm⁻¹.

### Exemple 2H : E-2-(p-méthoxy-cinnamyl)-1-cyclopropylméthyl-pipéridine (f. I ; Ar = p-méthoxy-phényle, m = 1, n = 2, R = cyclopropyle)

Le composé est préparé tel que décrit à l'exemple 1.1 à partir de E-2-(*p*-méthoxy-cinnamyl)-pipéridine (préparation 2H).
- Stade a E-2-(*p*-méthoxy-cinnamyl)-1-cyclopropane-carbonyl-pipéridine (f. II ; Ar = p-méthoxy-phényle, m = 1, n = 2, R = cyclopropyle). Rdt : 90 %.
   - - CCM :: Rf = 0,90- 1,00 (dichlorométhane/MeOH ammoniacal à 10 % 90/10 v/v.
- Stade b) E-2-(*p*-méthoxy-cinnamyl)-1-cyclopropylméthyl-pipéridine. Rdt : 72 %.
   - - CMM :: Rf = 0,80- 1,00 (dichlorométhane/MeOH ammoniacal à 10 % 90/10 v/v.

On introduit une étape finale de purification par chromato-flash sur colonne de silice en éluant par le mélange dichlorométhane/ méthanol ammoniacal à 10 % 95/5 v/v.
- - RMN :: 0,10- 0,30 (m, 2H) ; 0,40- 0,70 (m, 2H) ; 0,70- 1,10 (m, 1H); 1,20- 2,00 (m, 6H) ; 2,00- 2,80 (m, 6H) ; 2,80- 3,20 (m, 1H) ; 3,75 (s, 3H) ; 5,90- 6,50 (m, 2H) ; 6,70- 7,00 (m, 2H) ; 7,20- 7,40 (m, 2H).
Analyse (C₁₉ H₂₇ NO) : % C, H, N, O conformes ;
- IR (NaCl) : 2800, 1600, 1500, 1450, 1260, 1170, 1050, 980, 820 cm⁻¹.

### Exemple 2I : E-2-(1-napht-1-yl-propen-3-yl)-1-cyclopropyl-méthyl-pipéridine (f. I ; Ar = napht-1-yle, m = 1, n = 2, R = cyclopropyle)

Le composé est préparé tel que décrit à l'exemple 1.1 à partir de E-2-(1-napht-1-yl-propen-3-yl)-pipéridine (préparation 2I).
- Stade a) E-2-(1-napht-1-yl-propen-3-yl)-1-cyclopropane-carbonyl-pipéridine (f. Il ; Ar = napht-1-yle, m = 1, n = 2, R = cyclopropyle). Rdt : 99 %.
- Stade b) E-2-(1-napht-1-yl-propen-3-yl)-1-cyclopropylméthyl-pipéridine. Rdt : 92 %.
   - - CCM :: Rf = 0,75 (dichlorométhane/MeOH ammoniacal à 10 % 90/10 v/v)
   - - RMN :: 0,00- 0,20 (m, 2H) ; 0,40- 0,60 (m, 2H) ; 0,80- 1,00 (m, 1H); 1,10-1,40 (m, 1H) ; 1,40- 1,90 (m, 5H) ; 2,30- 2,80 (m, 6H) ; 3,00- 3,20 (m, 1H) ; 6,10- 6,30 (m, 1H) ; 7,10 (d, 1H) ; 7,30- 7,60 (m,4H); 7,70-7,90 (m, 2H) ; 8,10 (d,lH).

**Chlorhydrate** : F = 118 °C (acétate d'éthyle).
- Analyse (C₂₂ H₂₈ Cl N) : % C, H, Cl, N conformes ;
- IR (KBr) : 3400, 2950, 2450, 1450, 1430, 1210, 1020, 980, 880, 500 cm⁻¹.

### Exemple 2J : E-2-(1-thien-2 -yl-propen-3-yl)-1-cyclopropyl-méthyl-pipéridine (f. I ; Ar = thien-2-yle, m = 1, n = 2, R = cyclopropyle)

Le composé est préparé tel que décrit à l'exemple 1.1 à partir de E-2-(1-thien-2-yl-propen-3-yl)-pipéridine (préparation 2J).
- Stade a E-2-(1-thien-2-yl-propen-3-yl)-1-cyclopropane-carbonyl-pipéridine (f. II ; Ar = thien-2-yle, m = 1, n = 2, R = cyclopropyle). Rdt : 96 %.
- Stade b) E-2-(1-thien-2-yl-propen-3-yl)-1-cyclopropylméthyl-pipéridine. Rdt : 76 %.
   - - CCM :: Rf = 0,8 (dichlorométhane/MeOH ammoniacal à 10 % 90/10 v/v)

   On introduit une étape de purification par chromato-flash sur colonne de silice en éluant par le mélange dichlorométhane/méthanol ammoniacal à 10 % 95/5 v/v.
   - - RMN :: 0,00- 0,20 (m, 2H) ; 0,50- 0,60 (m, 2H) ; 0,80- 0,90 (m, 1H); 1,20- 1,80 (m, 6H) ; 2,20- 2,60 (m, 6H) ; 3,00- 3,10 (m, 1H) ; 6,00- 6,10 (m, 1H) ; 6,50 (d, 1H) ; 6,80- 7,00 (m, 2H); 7,10 (d, 1H).

**Chlorhydrate** : F = 175- 177 °C (acétate d'éthyle).
- Analyse (C₁₆ H₂₄ Cl N S) : % C, H, Cl, N, S conformes ;
- IR : 2980, 2500, 1470, 1210, 980, 830, 740, 560 cm⁻¹.

### Exemple 2K : Z-2-cinnamyl-1-cyclopropylméthyl-pyrrolidine (f. I- Z ; Ar = phényle, m = 1, n = 1, R = cyclopropyle)

Le composé est préparé tel que décrit à l'exemple 1.1 à partir de Z-2-cinnamyl-pyrrolidine (préparation 2K).
- Stade a) Z-2-cinnamyl-1-cyclopropane-carbonyl-pyrrolidine (f. II ; Ar = phényle, m = 1, n = 1, R = cyclopropyle). Rdt : 87 %.
   - - CCM :: Rf = 0,35- 0,55 (dichlorométhane/acétone 95/5 v/v)
- Stade b) Z-2cinnamyl-lcyclopropylméthyl-pyrrolidine. Rdt : 97 %.
   On introduit une étape de purification par chromato-flash sur colonne de silice en éluant par le mélange dichlorométhane/méthanol ammoniacal à 10 % 96/4 v/v.
   - - CCM :: Rf = 0,50- 0,80 (dichlorométhane/méthanol ammoniacal à 10 % 98/2 v/v)
   - - RMN :: 0,00- 0,20 (m, 2H) ; 0,40- 0,60 (m, 2H) ; 0,80- 0,90 (m, 1H); 1,50- 2,00 (m, 5H) ; 2,10- 2,20 (m, 1H) ; 2,30- 2,40 (m, 2H) ; 2,60- 2,80 (m, 2H) ; 3,80- 3,90 (m, 1H) ; 5,60- 5,70 (m, 1H) ; 6,50 (s, 1H); 7,10-7,30 (m, 5H).

**Chlorhydrate** : F = 129 °C (éther éthylique/isopropanol).
- Analyse (C₁₇ H₂₄ Cl N) : % C, H, Cl, N conformes ;
- IR (KBr) : 2900, 2480, 1440, 1180, 1060, 940, 800, 700, 500 cm⁻¹.

### Exemple 2L : E-2-(p-fluoro-cinnamyl)-1-cyclopropylméthyl-pyrrolidine (f. I ; Ar = p-fluorophényle, m = 1, n = 1, R = cyclopropyle)

Le composé est préparé tel que décrit à l'exemple 1.1 à partir de E-2-(*p*-fluoro-cinnamyl)-pyrrolidine (préparation 2L).
- Stade a) E-2-(*p*-fluoro-cinnamyl)-1-cyclopropane-carbonyl-pyrrolidine. (f. II ; Ar = *p*-fluoro-phényle, m = 1, n = 2, R = cyclopropyle). Rdt : 100 %.
- CCM : Rf = 0,40- 0,55 (dichlorométhane/acétone 95/5 v/v)
- Stade b) E-2-(*p*-fluoro-cinnamyl)-1-cyclopropylméthyl-pyrrolidine. Rdt : 84 %.
   - - CCM :: Rf = 0,75- 0,95 (dichlorométhane/MeOH ammoniacal à 10 % 90/10 v/v)
   - - RMN :: 0,10- 0,20 (m, 2H) ; 0,40- 0,60 (m, 2H) ; 0,90- 1,00 (m, 1H); 1,50- 2,10 (m, 5H) ; 2,10- 2,30 (m, 2H) ; 2,30- 2,40 (m, 1H) ; 2,50- 2,60 (m, 1H) ; 2,70- 2,80 (m, 1H) ; 3,30- 3,40 (m, 1H) ; 6,00- 6,20 (m, 1H) ; 6,40 (d,1H) ; 6,90- 7,00 (m, 2H) ; 7,20- 7,30 (m, 2H).
   - - IR (NaCl) :: 2950, 2850, 1600, 1500, 1220, 1160, 980, 840 cm⁻¹.

Chlorhydrate : Le chlorhydrate est très hygroscopique.
- Analyse (C₁₇ H₂₃ Cl F N) : % C, H, F, N conformes.

### Etudes pharmacologiques

Les composés de l'invention (I) et leurs sels montrent leur capacité d'interaction avec les récepteurs sigma dans des expériences de fixation, ou binding, réalisées *in vitro* en présence d'un ligand marqué spécifique des récepteurs sigma, le (+) [³H]-SKF 10,047. Le binding aux récepteurs de la phénylcyclidine (récepteurs PCP) réalisé en présence d'un ligand spécifique des récepteurs PCP, le [³H]-TCP, a permis d'étudier l'interaction éventuelle et indésirable des composés de l'invention avec ces récepteurs.

Par ailleurs, *in vivo,* la capacité des composés de l'invention (I) à inhiber les ulcères gastroduodénaux provoqués par administration de cystéamine a été mise en évidence et ce, de façon très favorable, par comparaison au chlorhydrate d'igmésine (DCI proposée), ou chlorhydrate de (+)-N-(cyclopropyl-méthyl)-1-éthyl-N-méthyl-1,4-diphénylbut-3-en-1-ylamine, composé préféré du brevet EP 362 001.

Egalement, la protection conférée par les produits de l'invention vis-à-vis de la diarrhée induite expérimentalement par une endotoxine bactérienne, le lipopolysaccharide de salmonelle, a été démontrée chez la souris.

### Etude in vitro

Les expériences de binding aux récepteurs sigma et PCP sont réalisées respectivement avec les ligands (+) [³H]-SKF 10,047 et [³H]-TCP, selon la technique décrite par Largent, B.L. et *al.* dans *J*. *Pharmacol. Exp. Ther.,* 1986, vol. 238, p. 739- 748. Le principe en est de mettre en compétition l'affinité du produit à l'essai et celle d'un ligand radioactif spécifique du récepteur étudié.

La technique consiste à incuber, dans des solutions de concentration variable du produit à l'essai, une préparation membranaire de cerveau de rat, préalablement chargée par le ligand marqué spécifique du récepteur étudié. Après filtration, on mesure la radioactivité de la solution, qui est représentative du déplacement de ce ligand marqué par le produit à l'essai. Les résultats sont exprimés en CI₅₀ du produit à l'essai, qui est la concentration permettant de déplacer le ligand tritié de 50 % de ses sites de liaison dans la préparation membranaire utilisée. La CI₅₀ est donc d'autant plus faible que l'affinité du produit pour le récepteur est grande. Les valeurs obtenues sont présentées au tableau 1 qui suit. L'halopéridol (DCI), connu entre autres propriétés pour son affinité aux récepteurs a, est présenté à titre de référence.

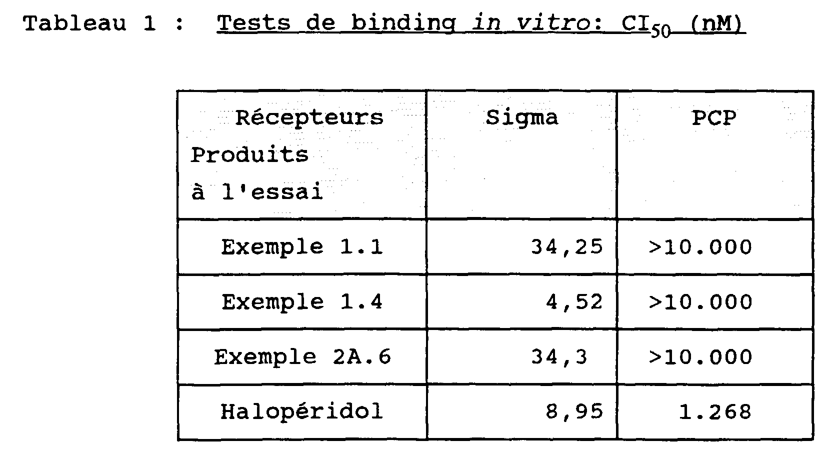

Ces résultats montrent que les produits (I) de l'invention ont une forte affinité pour les récepteurs sigma, notamment en ce qui concerne le composé de l'exemple 1.4. De plus, en comparaison avec l'halopéridol, les composés (I) de l'invention montrent une affinité que l'on peut considérer comme nulle pour les récepteurs PCP.

Dans une deuxième série d'expériences, on a mesuré pour d'autres produits de l'invention, la CI₅₀ vis-à-vis du [³H]-SKF 10,047 sur membranes de cerveau de rat, et comparé cette valeur à la CI₅₀ de l'halopéridol par le rapport CI₅₀ de l'halopéridol sur CI₅₀ du produit à tester. Les résultats figurent au tableau 2.

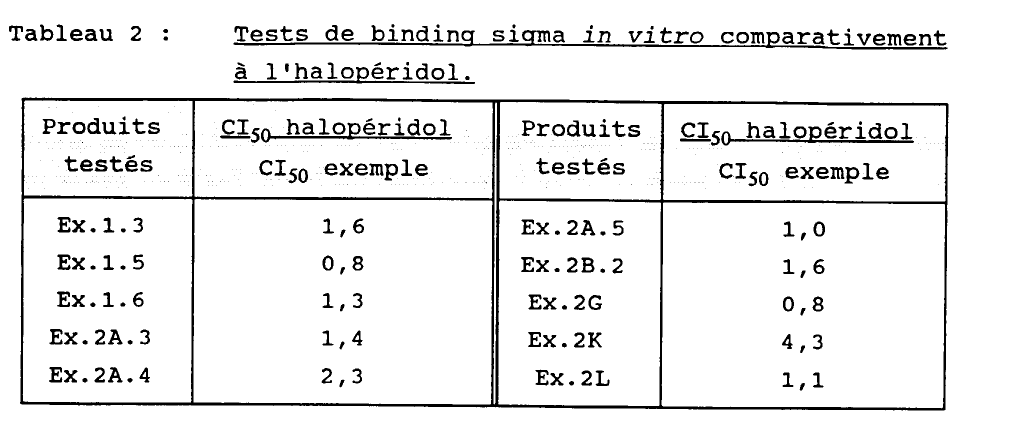

Ces résultats confirment que les produits (I) de l'invention ont une forte affinité pour les récepteurs sigma caractérisés par le [³H]-SKF 10,047, supérieure ou équivalente à celle de l'halopéridol, et, dans un certain nombre de cas, environ 2 à 4 fois plus forte.

En conclusion, ces résultats sont probants d'une affinité élevée des produits de l'invention pour les récepteurs sigma. De plus, cette affinité s'accompagne d'une spécificité remarquable puisque, contrairement à l'halopéridol, il n'y a pas d'interférence avec les récepteurs PCP.

### Etude in vivo : ulcère gastroduodénal provoqué par la cystéamine

L'activité des composés de l'invention sur le tractus gastro-intestinal a été montrée chez le rat par leur aptitude à inhiber les ulcères gastroduodénaux provoqués par l'administration de cystéamine. Pratiquement, l'étude est réalisée d'après la méthode décrite par Robert A. et coll., *Digestion,* 1974, vol. 11, p. 199- 214, sur des lots de rats mâles Sprague-Dawley d'un poids moyen de 200 g auxquels on administre par injection sous-cutanée une solution de chlorhydrate de cystéamine à raison de 400 mg/kg. Les produits à tester sont administrés aux animaux avant l'agent ulcérogène, respectivement 1 h ou 30 min selon qu'on utilise la voie d'administration orale ou intrapéritonéale. Dix-huit heures après, les rats sont sacrifiés par élongation ; leur estomac et duodénum sont prélevés, rincés avec du soluté physiologique, et épinglés sur un carton. La présence d'ulcères de la zone antro-pyloroduodénale est recherchée, et leur surface, exprimée en mm², est évaluée en multipliant les deux axes perpendiculaires principaux de la lésion. L'analyse statistique des résultats est réalisée par un test de Student portant sur les surfaces ulcérées, comparativement à un lot témoin. Les résultats obtenus après administration intra-péritonéale sont présentés au tableau 3, et exprimés en DE₅₀, qui est la dose efficace (mg/kg) de produit pour inhiber 50 % des ulcères provoqués par la cystéamine. L'igmésine est présentée à titre comparatif.

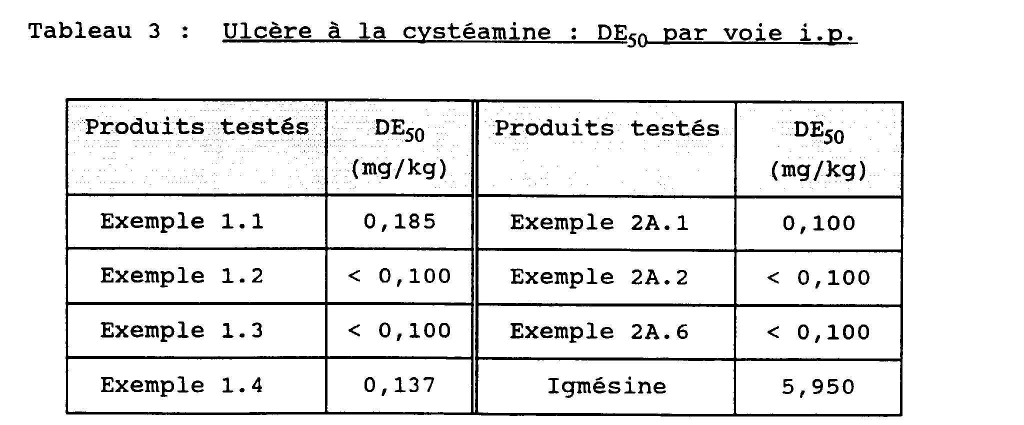

De façon probante, les composés de l'invention se montrent environ 30 à plus de 60 fois plus actifs que ce composé de l'art antérieur auquel ils sont comparés.

### Diarrhée induite par le lipopolysaccharide de salmonelle.

L'activité des produits de l'invention dans un modèle de diarrhée sécrétoire induite par le lipopolysaccharide (LPS) de *Salmonella enteritidis* a été étudiée selon un protocole inspiré de Ciancio M.J. et coll. *Gastroenterology,* 1992, vol. 103, p. 1437- 1443. A des souris mâles dBA₂, d'un poids compris entre 20 et 25 g, placées dans des cages individuelles à fond grillagé, le produit à l'essai est administré par voie orale, 1 h avant l'injection de LPS de S.enteridis (réf. L6761 Sigma) à la dose de 15 mg/kg i.v. On place un papier filtre prépesé sous chaque cage pour peser les fèces éliminées en 2 h. On calcule la DE₅₀ qui est la dose permettant de réduire de 50 % l'augmentation de fèces induite par le LPS comparativement à un lot contrôle ne recevant que l'endotoxine. Dans ce modèle, les produits (I) de l'invention testés ont montré une activité particulièrement intéressante, avec des DE₅₀ généralement inférieures à 100 µg/kg. Ces résultats indiquent l'utilisation possible des produits de l'invention pour le traitement symptomatique des diarrhées à composante sécrétoire, et d'étiologies diverses : toxique, infectieuse y compris virale, inflammatoire, post-antibiotique, ainsi que les diarrhées consécutives à une atteinte organique de la muqueuse.

La toxicité aiguë des produits de l'invention a été recherchée après administration par voie orale chez le rat. Ceci a permis de déterminer une valeur approchée de leur DL₅₀, qui est la dose létale pour 50 % des animaux dans les conditions de l'expérience. A des doses plus de cent fois supérieures à leur dose physiologiquement active, cette toxicité a été considérée comme négligeable.

### Formulations

Telles que décrites, ces propriétés pharmacologiques associées à la faible toxicité des composés de l'invention permettent d'envisager leur utilité sous forme de médicaments pour la prévention ou le traitement *i*) de dysfonctionnements neurologiques, notamment les états psychotiques, les états dépressifs, les troubles de la mémoire et du comportement, le stress et l'anxiété, ainsi que ii) de dysfonctionnements du tractus gastro-intestinal comme par exemple certaines formes d'ulcères gastroduodénaux, ou de diarrhées, notamment à composante sécrétoire.

De façon courante les posologies sont comprises de 0,1 à 1000 mg et plus particulièrement de 1 à 500 mg de produit selon la nature et la gravité de l'affection à traiter. Ces doses thérapeutiques journalières peuvent être réparties en plusieurs prises. De façon générale une posologie journalière de 5 mg à 250 mg de produit, répartis en deux à quatre prises, conduit à un résultat thérapeutique satisfaisant.

L'administration des produits de l'invention aux patients à traiter est réalisée sous la forme de médicaments de nature adaptée à l'affection à soigner.

Selon les cas, les préparations médicamenteuses seront, comme exemples non limitatifs, des comprimés, dragées, capsules, poudres, solutions, suspensions, gels ou suppositoires. Ces diverses formes pharmaceutiques sont préparées à partir des produits sous forme de base ou de leurs sels, et selon des méthodes couramment mises en oeuvres dans la pratique pharmaceutique.

Généralement dans les formes médicamenteuses de nature solide, le principe actif représente de 2 à 50 % en poids du total de la forme terminée alors que les excipients représentent de 98 à 50 %. Pour les formes liquides, ou pouvant être considérées comme telles, la quantité de principe actif est comprise entre 0,1 et 10 % en poids de la forme terminée alors que les excipients peuvent représenter de 99,9 à 90 % en poids de cette forme.

A titre d'illustration il est décrit la formule et la préparation de comprimés et de soluté isotonique avec le composé de l'exemple 1.4.

### Comprimés

### Formule :

- principe actif (composé de l'exemple 1.4) 5,0 à 25,0 mg
- polyvinylpyrrolidone 20,0 mg
- carboxyméthylamidon 8,0 mg
- stéarate de magnésium 2,0 mg
- silice colloïdale 0,4 mg
- lactose en quantité suffisante pour 200,0 mg

### Préparation :

Le principe actif en solution hydroalcoolique est mélangé au lactose, puis granulé avec la polyvinylpyrrolidone également en solution. Les grains sont séchés et tamisés sur une grille d'ouverture de 1 mm. Le carboxyméthylamidon est mélangé à la silice colloïdale, puis ajouté aux granulés. On mélange ensuite intimement avec le stéarate de magnésium, puis comprime à raison de 200,0 mg par comprimé.

### Soluté isotonique injectable

### Formule :

- substance active (I), chlorhydrate de l'Ex. 1.4 10,0 mg
- chlorure de sodium 9,0 mg
- eau distillée en quantité suffisante pour 1,0 ml

### Préparation :

Le soluté isotonique est réparti en ampoules de volume approprié qui sont, après scellement, stérilisées par des moyens thermiques habituels ; ou bien le soluté est stérilisé par filtration, réparti en ampoules qui sont ensuite scellées, l'ensemble de ces opérations étant réalisé sous atmosphère stérile.

## Revendications

1. 2-arylalkényl-azacycloalkanes de formule (I) 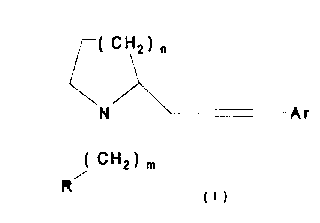 dans laquelle :
Ar est aryle ou hétéroaryle dont l'hétéroatome est l'azote et/ou le soufre, optionnellement mono à trisubstitué, par des halogènes, des radicaux, alkyle inférieur, haloalkyle inférieur ou alkoxy inférieur dont la chaîne carbonée comprend de 1 à 4 atomes de carbone,
m a pour valeur 1 ou 2,
n a pour valeur 1 à 3,
R est phényle ou cycloalkyle comprenant de 3 à 7 atomes de carbone,
leurs isomères, leurs dérivés dans lesquels un atome est remplacé par un de ses isotopes radioactifs, et leurs sels d'addition avec les acides pharmaceutiquement acceptables.

2. Composés selon la revendication 1 caractérisés en ce que Ar représente un radical phényle optionnellement mono à disubstitué par des halogènes ou des radicaux - alkyle inférieur, haloalkyle inférieur, alkoxy inférieur, dont la chaîne carbonée comprend de 1 à 4 atomes de carbone.

3. Composés selon l'une des revendications 1 ou 2 caractérisés en ce que n a pour valeur 1 ou 2.

4. Composés selon l'une des revendications 1 à 3 caractérisés en ce que R est cyclopropyle ou cyclobutyle.

5. Composés selon la revendication 1 choisis parmi le groupe comprenant :
(+/-)-E-2-cinnamyl-1-cyclopropylméthylpyrrolidine
(+)-E-2-cinnamyl-1-cyclopropylméthylpyrrolidine
(-)-E-2-cinnamyl-1-cyclopropylméthylpyrrolidine
E-2-cinnamyl-1-cyclobutylméthylpyrrolidine
E-2-cinnamyl-1-cyclopropyléthyl-pyrrolidine
E-2-cinnamyl-1-phénéthyl-pyrrolidine
E-2-cinnamyl-1-cyclopropylméthylpipéridine
(+)-E-2-cinnamyl-1-cyclopropylméthyl-pipéridine
(-)-E-2-cinnamyl-cyclopropylméthyl-pipéridine
E-2-cinnamyl-1-cyclobutylméthylpipéridine
E-2-cinnamyl-1-phénéthyl-pipéridine
Z-2-cinnamyl-1-cyclopropylméthylpipéridine
E-2-(*p*-fluoro-cinnamyl)-1-cyclopropylméthyl-pyrrolidine
Z-2-(*p*-fluoro-cinnamyl)-1-cyclopropylméthyl-pipéridine
E-2-(*p*-chloro-cinnamyl)-1-cyclopropylméthyl-pipéridine
E-2-(*m*-chloro-cinnamyl)-1-cyclopropylméthyl-pipéridine
E-2-(3,4-dichloro-cinnamyl)-1-cyclopropylméthyl-pipéridine
E-2-(*p*-méthyl-cinnamyl)-1-cyclopropylméthyl-pipéridine
E-2-(*p*-méthoxy-cinnamyl)-1-cyclopropylméthyl-pipéridine
E-2-(1-napht-1-yl-propen-3-yl)-1-cyclopropylméthyl-pipéridine
E-2-(1-thien-2-yl-propen-3-yl)-1-cyclopropylméthyl-pipéridine
Z-2-cinnamyl-1-cyclopropylméthyl-pyrrolidine
E-2-(*p*-fluoro-cinnamyl)-1-cyclopropylméthyl-pyrrolidine.

6. Composé selon la revendication 5, choisi parmi le groupe comprenant :
(+/-)-E-2-cinnamyl-1-cyclopropylméthylpyrrolidine
(-)-E-2-cinnamyl-1-cyclopropylméthylpyrrolidine
E-2-cinnamyl-1-cyclopropyléthyl-pyrrolidine
E-2-cinnamyl-1-phénéthyl-pyrrolidine
E-2-cinnamyl-1-cyclopropylméthylpipéridine
E-2-cinnamyl-1-cyclobutylméthylpipéridine
E-2-(*p*-fluoro-cinnamyl)-1-cyclopropylméthyl-pyrrolidine
E-2-(*p*-chloro-cinnamyl)-1-cyclopropylméthyl-pipéridine
E-2-(*m*-chloro-cinnamyl)-1-cyclopropylméthyl-pipéridine
E-2-(3,4-dichloro-cinnamyl)-1-cyclopropylméthyl-pipéridine
E-2-(*p*-méthoxy-cinnamyl)-1-cyclopropylméthyl-pipéridine
E-2-(1-napht-1-yl-propen-3-yl)-1-cyclopropylméthyl-pipéridine.

7. Composé selon la revendication 1 E-2-(3,4-dichloro-cinnamyl)-1-cyclopropylméthyl-pipéridine.

8. Médicament destiné à la prévention ou au traitement de la diarrhée, caractérisé en ce qu'il comprend un 2-arylalkényl-azacycloalkane (I) selon l'une des revendications 1 à 7.

9. Médicament selon la revendication 8, caractérisé en ce que le 2-arylalkényl-azacycloalkane est le E-2-(3,4-dichloro-cinnamyl)-1-cyclopropylméthyl-pipéridine.

10. Utilisation d'un composé selon l'une quelconque des revendications 1 à 7 pour la préparation d'un médicament destiné au traitement de la diarrhée.

11. Utilisation selon la revendication 10, caractérisée en ce que le composé est le E-2-(3,4-dichloro-cinnamyl)-1-cyclopropylméthyl-pipéridine.

12. Procédé de préparation d'un 2-arylalkényl-azacycloalkane (I) défini à la revendication 1, caractérisé en ce qu'il consiste :
- à acyler un intermédiaire azacycloalkane (III) 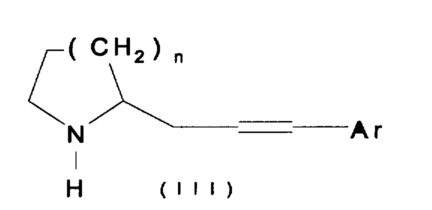
dans lequel n et Ar sont tels que définis pour (I), par un
réactif (IV) : 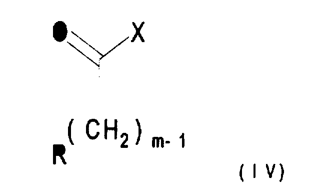 dans lequel m et R sont tels que définis pour (I), et X est -OH ou un halogène comme le chlore ou le brome,
pour obtenir un dérivé carboxamide intermédiaire (II) 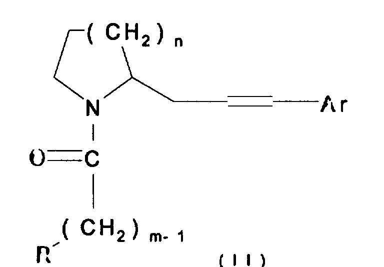
- puis à réduire le carboxamide (II) par un hydrure métallique ou organométallique dérivé de préférence de l'aluminium.

## Patentansprüche

1. 2-Arylalkenylazacycloalkane der Formel (I) 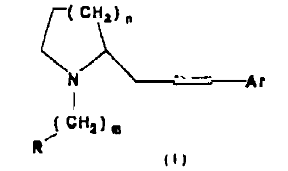 worin:
Ar für gegebenenfalls ein- bis dreifach durch Halogen oder Niederalkyl-, Halogenniederalkyl- oder Niederalkoxyreste mit 1 bis 4 Kohlenstoffatomen in der Kohlenstoffkette substituiertes Aryl oder Heteroaryl mit Stickstoff und/oder Schwefel als Heteroatom steht,
m einen Wert von 1 oder 2 hat,
n einen Wert von 1 bis 3 hat,
R für Phenyl oder Cycloalkyl mit 3 bis 7 Kohlenstoffatomen steht,
deren Isomere, deren Derivate, in denen ein Atom durch eines seiner radioaktiven Isotope ersetzt ist, und deren Additionssalze mit pharmazeutisch unbedenklichen Säuren.

2. Verbindungen nach Anspruch 1, dadurch gekennzeichnet, daß Ar für einen gegebenenfalls ein- oder zweifach durch Halogen oder Niederalkyl-, Halogenniederalkyl- oder Niederalkoxyreste mit 1 bis 4 Kohlenstoffatomen in der Kohlenstoffkette substituierten Phenylrest steht.

3. Verbindungen nach Anspruch 1 oder 2, dadurch gekennzeichnet, daß n einen Wert von 1 oder 2 hat.

4. Verbindungen nach einem der Ansprüche 1 bis 3, dadurch gekennzeichnet, daß R für Cyclopropyl oder Cyclobutyl steht.

5. Verbindungen nach Anspruch 1 aus der Gruppe bestehend aus:
(+/-)-E-2-Cinnamyl-1-cyclopropylmethylpyrrolidin
(+)-E-2-Cinnamyl-l-cyclopropylmethylpyrrolidin
(-)-E-2-Cinnamyl-1-cyclopropylmethylpyrrolidin
E-2-Cinnamyl-1-cyclobutylmethylpyrrolidin
E-2-Cinnamyl-1-cyclopropylethylpyrrolidin
E-2-Cinnamyl-1-phenethylpyrrolidin
E-2-Cinnamyl-1-cyclopropylmethylpiperidin
(+)-E-2-Cinnamyl-1-cyclopropylmethylpiperidin
(-)-E-2-Cinnamyl-cyclopropylmethylpiperidin
E-2-Cinnamyl-1-cyclobutylmethylpiperidin
E-2-Cinnamyl-1-phenethylpiperidin
Z-2-Cinnamyl-1-cyclopropylmethylpiperidin
E-2-(*p*-Fluorcinnamyl)-1-cyclopropylmethylpyrrolidin
Z-2-(*p*-Fluorcinnamyl)-1-cyclopropylmethylpiperidin
E-2-(*p*-Chlorcinnamyl)-1-cyclopropylmethylpiperidin
E-2-(m-Chlorcinnamyl)-1-cyclopropylmethylpiperidin
E-2-(3,4-Dichlorcinnamyl)-1-cyclopropylmethylpiperidin
E-2-(*p*-Methylcinnamyl)-1-cyclopropylmethylpiperidin
E-2-(*p*-Methoxycinnamyl)-1-cyclopropylmethylpiperidin
E-2-(1-Naphth-1-ylpropen-3-yl)-1-cyclopropylmethyl-piperidin
E-2-(1-Thien-2-ylpropen-3-yl)-1-cyclopropylmethyl-piperidin
Z-2-Cinnamyl-1-cyclopropylmethylpyrrolidin
E-2-(*p*-Fluorcinnamyl)-1-cyclopropylmethylpyrrolidin.

6. Verbindung nach Anspruch 5 aus der Gruppe bestehend aus:
(+/-)-E-2-Cinnamyl-1-cyclopropylmethylpyrrolidin
(-)-E-2-Cinnamyl-1-cyclopropylmethylpyrrolidin
E-2-Cinnamyl-1-cyclopropylethylpyrrolidin
E-2-Cinnamyl-1-phenethylpyrrolidin
E-2-Cinnamyl-1-cyclopropylmethylpiperidin
E-2-Cinnamyl-1-cyclobutylmethylpiperidin
E-2-(*p*-Fluorcinnamyl)-1-cyclopropylmethylpyrrolidin
E-2-(*p*-Chlorcinnamyl)-1-cyclopropylmethylpiperidin
E-2-(*m*-Chlorcinnamyl)-1-cyclopropylmethylpiperidin
E-2-(3,4-Dichlorcinnamyl)-1-cyclopropylmethylpiperidin
E-2-(*p*-Methoxycinnamyl)-1-cyclopropylmethylpiperidin
E-2-(1-Naphth-1-ylpropen-3-yl)-1-cyclopropylmethyl-piperidin.

7. Verbindung nach Anspruch 1, bei der es sich um E-2-(3,4-Dichlorcinnamyl)-1-cyclopropylmethylpiperidin handelt.

8. Arzneimittel zur Prävention oder Behandlung von Diarrhöe, dadurch gekennzeichnet, daß es ein 2-Arylalkenylazacycloalkan (I) nach einem der Ansprüche 1 bis 7 enthält.

9. Arzneimittel nach Anspruch 8, dadurch gekennzeichnet, daß es sich bei dem 2-Arylalkenylazacycloalkan um E-2-(3,4-Dichlorcinnamyl)-1-cyclopropylmethylpiperidin handelt.

10. Verwendung einer Verbindung nach einem der Ansprüche 1 bis 7 zur Herstellung eines Arzneimittels zur Behandlung von Diarrhöe.

11. Verwendung nach Anspruch 10, dadurch gekennzeichnet, daß es sich bei der Verbindung um E-2-(3,4-Dichlorcinnamyl)-1-cyclopropylmethylpiperidin handelt.

12. Verfahren zur Herstellung eines 2-Arylalkenylazacycloalkans (I) gemäß Anspruch 1, dadurch gekennzeichnet, daß man:
- ein Azacycloalkan-Zwischenprodukt (III) 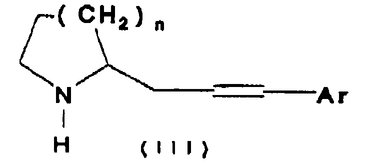 worin n und Ar die unter (I) angegebene Bedeutung besitzen, mit einem Reagenz (IV) 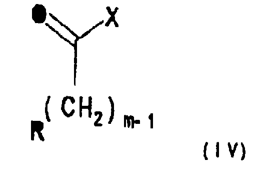 worin m und R die unter (I) angegebene Bedeutung besitzen und X für -OH oder ein Halogen wie Chlor oder Brom steht,
zu einem Carbonsäureamid-Derivat (II) 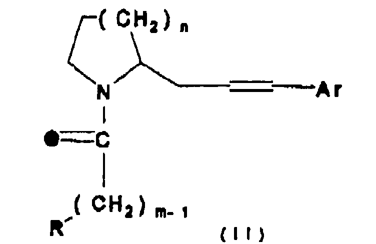 acyliert und danach
- das Carbonsäureamid (II) mit einem Metallhydrid oder Organometallhydrid, vorzugsweise abgeleitet von Aluminium, reduziert.

## Claims

1. 2-Arylalkenyl-azacycloalkanes of formula (I) 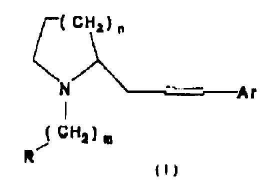 in which:
Ar is aryl or heteroaryl in which the hetero atom is nitrogen and/or sulphur, optionally mono- to trisubstituted, with halogens or lower alkyl, lower haloalkyl or lower alkoxy radicals in which the carbon-based chain comprises from 1 to 4 carbon atoms,
m is 1 or 2,
n is 1 to 3,
R is phenyl or cycloalkyl, comprising from 3 to 7 carbon atoms,
their isomers, their derivatives in which an atom is replaced with one of its radioactive isotopes, and their addition salts with pharmaceutically acceptable acids.

2. Compounds according to Claim 1, characterized in that Ar represents a phenyl radical optionally mono- or disubstituted with halogens or lower alkyl, lower haloalkyl or lower alkoxy radicals, in which the carbon-based chain comprises from 1 to 4 carbon atoms.

3. Compounds according to either of Claims 1 and 2, characterized in that n is 1 or 2.

4. Compounds according to one of Claims 1 to 3, characterized in that R is cyclopropyl or cyclobutyl.

5. Compounds according to Claim 1, chosen from the group comprising:
(±)-E-2-cinnamyl-1-cyclopropylmethylpyrrolidine
(+)-E-2-cinnamyl-1-cyclopropylmethylpyrrolidine
(-)-E-2-cinnamyl-1-cyclopropylmethylpyrrolidine
E-2-cinnamyl-l-cyclobutylmethylpyrrolidine
E-2-cinnamyl-1-cyclopropylethylpyrrolidine
E-2-cinnamyl-1-phenethylpyrrolidine
E-2-cinnamyl-1-cyclopropylmethylpiperidine
(+)-E-2-cinnamyl-1-cyclopropylmethylpiperidine
(-)-E-2-cinnamyl-1-cyclopropylmethylpiperidine
E-2-cinnamyl-1-cyclobutylmethylpiperidine
E-2-cinnamyl-1-phenethylpiperidine
Z-2-cinnamyl-1-cyclopropylmethylpiperidine
E-2-(*p*-fluorocinnamyl)-1-cyclopropylmethylpyrrolidine
Z-2-(*p*-fluorocinnamyl)-1-cyclopropylmethylpiperidine
E-2-(*p*-chlorocinnamyl)-1-cyclopropylmethylpiperidine
E-2-(*m*-chlorocinnamyl)-1-cyclopropylmethylpiperidine
E-2-(3,4-dichlorocinnamyl)-1-cyclopropylmethyl-piperidine
E-2-(*p*-methylcinnamyl)-1-cyclopropylmethylpiperidine
E-2-(*p*-methoxycinnamyl)-1-cyclopropylmethylpiperidine
E-2-(1-naphth-1-ylpropen-3-yl)-1-cyclopropylmethyl-piperidine
E-2-(1-thien-2-ylpropen-3-yl)-1-cyclopropylmethyl-piperidine
Z-2-cinnamyl-1-cyclopropylmethylpyrrolidine
E-2-(*p*-fluorocinnamyl)-1-cyclopropylmethylpyrrolidine.

6. Compound according to Claim 5, chosen from the group comprising:
(±)-E-2-cinnamyl-1-cyclopropylmethylpyrrolidine
(-)-E-2-cinnamyl-1-cyclopropylmethylpyrrolidine
E-2-cinnamyl-1-cyclopropylethylpyrrolidine
E-2-cinnamyl-1-phenethylpyrrolidine
E-2-cinnamyl-1-cyclopropylmethylpiperidine
E-2-cinnamyl-1-cyclobutylmethylpiperidine
E-2-(*p*-fluorocinnamyl)-1-cyclopropylmethylpyrrolidine
E-2-(*p*-chlorocinnamyl)-1-cyclopropylmethylpiperidine
E-2-(*m*-chlorocinnamyl)-1-cyclopropylmethylpiperidine
E-2-(3,4-dichlorocinnamyl)-1-cyclopropylmethyl-piperidine
E-2-(*p*-methoxycinnamyl)-1-cyclopropylmethylpiperidine
E-2-(1-naphth-1-ylpropen-3-yl)-1-cyclopropylmethyl-piperidine.

7. Compound according to Claim 1, E-2-(3,4-dichlorocinnamyl)-1-cyclopropylmethylpiperidine.

8. Medicinal product intended for the prevention or treatment of diarrhoea, characterized in that it comprises a 2-arylalkenyl-azacycloalkane (I) according to one of Claims 1 to 7.

9. Medicinal product according to Claim 8, characterized in that the 2-arylalkenyl-azacycloalkane is E-2-(3,4-dichlorocinnamyl)-1-cyclopropylmethyl-piperidine.

10. Use of a compound according to any one of Claims 1 to 7, for the preparation of a medicinal product intended for the treatment of diarrhoea.

11. Use according to Claim 10, characterized in that the compound is E-2-(3,4-dichlorocinnamyl)-1-cyclopropylmethylpiperidine.

12. Process for preparing a 2-arylalkenyl-azacycloalkane (I) defined in Claim 1, characterized in that it consists:
- in acylating an intermediate azacycloalkane (III) 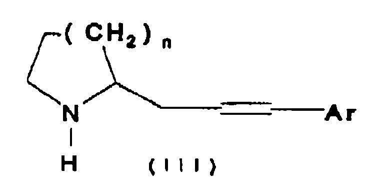 in which n and Ar are as defined for (I), with a reagent (IV): 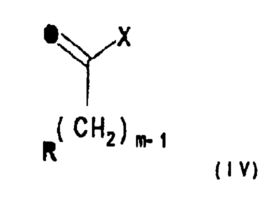 in which m and R are as defined for (I) and X is -OH or a halogen such as chlorine or bromine,
to give an intermediate carboxamide derivative (II) 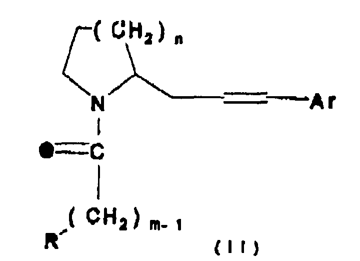
- and then in reducing the carboxamide (II) with a metal hydride or an organometallic hydride preferably derived from aluminium.
